# EUROPEAN PATENT APPLICATION

(11) **EP 4 070 857 A1**
(43) Date of publication of application: **12.10.2022**
(21) Application number: 20895824.9
(22) Date of filing: 01.12.2020
(51) Int. Cl.: A61Q 5/12, A61K 8/34, A61K 8/39, A61K 8/41, A61K 8/44, A61K 8/898

(54) **HAIR COSMETIC COMPOSITION**

(30) Priority: 05.12.2019 JP 2019220608; 20.11.2020 JP 2020193089
(71) Applicant: Kao Corporation, Chuo-ku Tokyo 103-8210 (JP)
(72) Inventor: WATANABE, Shunichi, Tokyo 131-8501 (JP); FUKUHARA, Chikako, Tokyo 131-8501 (JP); YOKOTA, Yuuki, Tokyo 131-8501 (JP); CHAN, Yungchen, Hsinchu Industrial Areas, Hukou Hsiang Hsinchu Hsien, 30352 (TW); WANG, Shufen, Hsinchu Industrial Areas, Hukou Hsiang Hsinchu Hsien, 30352 (TW); PHOOPICHAYANUN, Chalouyluk, Klongtoey, Bangkok 10110 (TH); SRIWONGSITANONT, Supaporn, Klongtoey, Bangkok 10110 (TH)
(74) Representative: Hoffmann Eitle
(86) International application number: PCT/JP2020/044740
(87) International publication number: WO 2021/112099

(57) **Abstract**

A hair cosmetic composition and a hair cosmetic using said hair cosmetic composition which are packed into a non-gas type foam dispensing container for use, wherein: said composition contains the following component (A), component (B), and component (C), and component (A) is a cationic surfactant, component (B) is an amphoteric surfactant, and component (C) is at least one component selected from the group consisting of nonionic surfactants (C1), modified silicones having a cationic group (C2), and sugar alcohols having a carbon number of at least 4 (C3); the mass ratio of [(B)/(A)] is 0.4-5, inclusive; and the higher alcohol content is no more than 0.1 mass%.

## Description

### FIELD OF THE INVENTION

The present invention relates to a hair cosmetic composition.

### BACKGROUND OF THE INVENTION

The hair is damaged by the living environment (e.g., ultraviolet rays and heat of the sunlight), the daily hair care activities (e.g., friction by hair washing or brushing), and the chemical treatment (e.g., coloring and perm). Accordingly, hair cosmetics that suppress the damage of the hair are important, and hair cosmetics that can achieve the efficient hair care activities without stress are being demanded.

In response to the demand, hair cosmetics using a non-gas type foam discharge container using no propellant have been proposed.

PTL 1 (JP 2017-128618 A) describes that non-gas type foamy hair cosmetics containing stearyltrimethylammonium chloride, polyoxyethylene-polyoxypropylene butyl ether, dipropylene glycol, and ethanol in the prescribed amounts can form highly elastic foam, and can impart excellent treatment feeling and the like to the hair and an appropriate feeling of foam without dripping.

PTL 2 (JP 2018-158901 A) describes that hair cosmetics containing a cationic surfactant, a fatty acid polyglyceryl having 8 to 18 carbon atoms having an HLB value of 12 to 18, polyoxyethylene alkyl glucoside, and one kind or two or more kinds of a polyhydric alcohol selected from di- or tripropylene glycol, 1,2-propanediol, and 1,3-propanediol are excellent to provide feeling of foam as applying, also flexibility, smoothness, and good-aligned of the finished hair without stickiness.

PTL 3 (JP 2018-2637 A) describes that hair cosmetics selected from a hair cleaning agent and an in-bath type hair protective agent included in a composition containing an amphoteric surfactant and a nonionic surfactant, housed in a non-gas type foamer container having three or more porous bodies with a mesh aperture in the prescribed ranges, is to be discharged in an amount of 3 mL or more at a time for preventing the friction and damages of hair during wash-off and after the use thereof.

PTL 4 (JP 2006-104149 A) describes that a hair rinse composition for a non-aerosol foam discharging container containing an amphoteric surfactant, a cationic surfactant, and a higher alcohol, wherein the amphoteric surfactant blended in an amount within the prescribed ranges, and a mass ratio of the amphoteric surfactant, the cationic surfactant, and the higher alcohol within the prescribed range, and a hair rinse product using this composition can be discharged as a stable foam and can be rinsed smoothly.

PTL 5 (JP 2001-10930 A) describes that non-aerosol foam hair cosmetics filled in a foam discharging container, containing a compound having an OH group with an IOB value within the prescribed range, a silicone oil and a surfactant, is excellent in foamability, being fine and creamy, and provides smoothness of hair after application.

### SUMMARY OF THE INVENTION

The present invention relates to the following items [1] and [2].
[1] A hair cosmetic composition
   containing or being prepared by blending a component (A), a component (B), and a component (C) below:
   component (A): a cationic surfactant,
   component (B): an amphoteric surfactant, and
   component (C): one or more kinds selected from the group consisting of a nonionic surfactant (C1), a modified silicone having a cationic group (C2), and a sugar alcohol having 4 or more carbon atoms (C3),
   having a mass ratio [(B)/(A)] of a content or a blending amount of the component (B) to a content or a blending amount of the component (A) of 0.4 or more and 5 or less,
   having a content or a blending amount of a higher alcohol of 0.1% by mass or less, and
   being used by filling up a non-gas type foam discharge container.
[2] Hair cosmetics including the hair cosmetic composition according to the item [1] filled up a non-gas type foam discharge container.

### BRIEF DESCRIPTION OF DRAWING

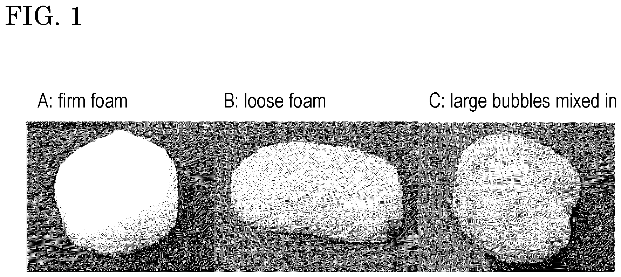

Fig. 1 is a set of photographs showing the state of the foam quality as the evaluation standard used in the evaluation of the foam quality.

### DETAILED DESCRIPTION OF THE INVENTION

In application of a hair cosmetic composition to the hair with a non-gas type foam discharge container, the hair cosmetic composition is mixed with air in the foam discharge container, and is extruded through a mesh and thus discharged in the form of foam. However, in discharging the hair cosmetic composition from the foam discharge container under a low temperature environment, such as -5°C, there is a problem including occurrence of pump failure. It has been found that the pump failure is due to the clogged mesh with the precipitate of the composition or the like since the hair cosmetic composition is poor to be stable under a low temperature environment. Accordingly, the enhancement of the storage stability under a low temperature environment is demanded for the hair cosmetic composition. On the other hand, the improvement of the storage stability under a low temperature environment may restrict the components blended in the hair cosmetic composition.

The techniques of the PTLs 1 to 5 fail to satisfy in the storage stability under a low temperature environment, the quality of foam, the capability to protect the hair against damages caused by friction, the smoothness imparted to the hair and the suppression of entanglement of the hair during time of rinsing, and thus the improvement thereof is being demanded.

The present invention relates to a hair cosmetic composition that has excellence in the storage stability under the low temperature environment, the quality of foam discharged from a foam discharge container, the capability to protect the hair against damages caused by friction, the favorable smoothness imparted to the hair and the suppression of entanglement of the hair during time of rinsing, and hair cosmetics using the hair cosmetic composition.

The present inventors have found that the problem can be solved by a hair cosmetic composition containing or being prepared by blending a cationic surfactant, an amphoteric surfactant, and one or more kinds selected from the group consisting of a nonionic surfactant, a modified silicone having a cationic group, and a sugar alcohol having 4 or more carbon atoms, having a mass ratio of the content or the blending amount of the amphoteric surfactant to the content or the blending amount of the cationic surfactant within the prescribed range, and having a content or a blending amount of a higher alcohol of 0.1% by mass or less.

Specifically, the present invention relates to the following items [1] and [2].
[1] A hair cosmetic composition
   containing or being prepared by blending a component (A), a component (B), and a component (C) below:
   component (A): a cationic surfactant,
   component (B): an amphoteric surfactant, and
   component (C): one or more kinds selected from the group consisting of a nonionic surfactant (C1), a modified silicone having a cationic group (C2), and a sugar alcohol having 4 or more carbon atoms (C3),
   having a mass ratio [(B)/(A)] of a content or a blending amount of the component (B) to a content or a blending amount of the component (A) of 0.4 or more and 5 or less,
   having a content or a blending amount of a higher alcohol of 0.1% by mass or less, and
   being used by filling up a non-gas type foam discharge container.
[2] Hair cosmetics including the hair cosmetic composition according to the item [1] filled up a non-gas type foam discharge container.

According to the present invention, a hair cosmetic composition that has excellence in the storage stability under the low temperature environment, the quality of foam discharged from a foam discharge container, the capability to protect the hair against damages caused by friction, the favorable smoothness imparted to the hair and the suppression of entanglement of the hair during time of rinsing, and hair cosmetics using the hair cosmetic composition can be provided.

### [Hair Cosmetic Composition]

The hair cosmetic composition of the present invention (which may be hereinafter referred simply to as a "hair cosmetic composition") contains or is prepared by blending a component (A), a component (B), and a component (C) below:
component (A): a cationic surfactant,
component (B): an amphoteric surfactant, and
component (C): one or more kinds selected from the group consisting of a nonionic surfactant (C1), a modified silicone having a cationic group (C2), and a sugar alcohol having 4 or more carbon atoms (C3),
has a mass ratio [(B)/(A)] of the content or the blending amount of the component (B) to the content or the blending amount of the component (A) of 0.4 or more and 5 or less,
has a content or a blending amount of a higher alcohol of 0.1% by mass or less, and
is used by filling up a non-gas type foam discharge container.

In the present invention, a storage stability at -5°C is used as an index of the "storage stability under a low temperature environment". With an excellent storage stability at -5°C, the hair cosmetic composition can be sufficiently used by filling up a non-gas type foam discharge container even under a use environment in the cold regions. In the following description, the "storage stability under a low temperature environment" may be referred simply to as a "low temperature stability".

The mechanism of the effects of the present invention exerted by the hair cosmetic composition of the present invention having the aforementioned configuration is not clear, but can be estimated as follows.

The hair cosmetic composition of the present invention contains or is prepared by blending a cationic surfactant as the component (A), an amphoteric surfactant as the component (B), and one or more kinds selected from the group consisting of a nonionic surfactant (C1), a modified silicone having a cationic group (C2), and a sugar alcohol having 4 or more carbon atoms (C3) as the component (C). It is considered that with a mass ratio [(B)/(A)] of the content or the blending amount of the component (B) to the content or the blending amount of the component (A) in the hair cosmetic composition within the prescribed range, the component (A) enhances the solubility of the component (B), and the low temperature stability is enhanced. It is considered that a mass ratio [(B)/(A)] within the prescribed range enhances the quality of foam discharged from a foam discharge container, easily integrating the foam onto the hair, the hair then can be protected against damages caused by friction of the hair.

It is also considered that since the hair cosmetic composition contains or is prepared by blending a higher alcohol incorporated in the content or the blending amount of the particular value or less, the components of the composition are not precipitated under a low temperature environment, and the low temperature stability is further enhanced.

It is also considered that the component (C) protects the hair against damages caused by friction, and imparts favorable smoothness to the hair and suppresses entanglement of the hair during time of rinsing.

### <Component (A): Cationic Surfactant>

The hair cosmetic composition contains or is prepared by blending a cationic surfactant as the component (A) (which may be hereinafter referred simply to as "(A)").

Examples of the cationic surfactant include (i) an alkyltrimethylammonium salt, (ii) an alkoxyalkyltrimethylammonium salt, (iii) a dialkyldimethylammonium salt, (iv) an alkylamidoalkyltrimethylammonium salt, (v) an alkyldimethylamine and a salt thereof, (vi) an alkoxyalkyldimethylamine and a salt thereof, and (vii) an alkylamidoalkyldimethylamine and a salt thereof.

Preferred examples of the alkyltrimethylammonium salt (i) include an alkyltrimethylammonium salt having an alkyl group having 12 or more and 22 or less, more preferably 16 or more and 20 or less, carbon atoms, and specific examples thereof include cetyltrimethylammonium chloride (cetrimonium chloride), stearyltrimethylammonium chloride (steartrimonium chloride), and behenyltrimethylammonium chloride.

Preferred examples of the alkoxyalkyltrimethylammonium salt (ii) include an alkoxyalkyltrimethylammonium salt having an alkoxy group having 12 or more and 22 or less, more preferably 16 or more and 20 or less, carbon atoms, and specific examples thereof include stearoxypropyltrimethylammonium chloride, stearoxyethyltrimethylammonium chloride, and stearoxyhydroxypropyltrimethylammonium chloride.

Preferred examples of the dialkyldimethylammonium salt (iii) include a dialkyldimethylammonium salt having an alkyl group having 12 or more and 22 or less, more preferably 16 or more and 20 or less, carbon atoms, and specific examples thereof include distearyldimethylammonium chloride.

Preferred examples of the alkylamidoalkyltrimethylammonium salt (iv) include an alkylamidoalkyltrimethylammonium salt having an alkyl group having 11 or more and 21 or less, more preferably 13 or more and 19 or less, carbon atoms, and specific examples thereof include palmitamidopropyltrimethylammonium chloride (palmitamidopropyltrimonium chloride).

The alkyldimethylamine (v), the alkoxyalkyldimethylamine (vi), and the alkylamidoalkyldimethylamine (vii) each reacts with an acid to form a tertiary amine salt, which functions as a cationic surfactant.

The alkyl group in the alkyldimethylamine and a salt thereof (v) and the alkoxyalkyldimethylamine and a salt thereof (vi) is preferably an alkyl group having 12 or more and 22 or less, more preferably 16 or more and 20 or less, carbon atoms.

The alkyl group in the alkylamidoalkyldimethylamine and a salt thereof (vii) is preferably an alkyl group having 11 or more and 21 or less, more preferably 15 or more and 19 or less, carbon atoms.

The amines (v) to (vii) each may be blended as a salt in the hair cosmetic composition after reacting with an acid in advance, or may be blended directly in the hair cosmetic composition, and an acid may be further blended in the hair cosmetic composition to form a salt in the composition. Accordingly, the amines and salts thereof herein are defined as cationic surfactants. As the content or the blending amount thereof, the mass of the amine is referred to.

Examples of the salts of the amines (v) to (vii) include salts with an organic acid or an inorganic acid. Examples of the organic acid include a monocarboxylic acid, such as acetic acid and a propionic acid; a dicarboxylic acid, such as malonic acid, succinic acid, glutaric acid, adipic acid, maleic acid, fumaric acid, and phthalic acid; a polycarboxylic acid, such as polyglutamic acid; a hydroxycarboxylic acid, such as glycolic acid, lactic acid, hydroxyacrylic acid, glyceric acid, malic acid, tartaric acid, and citric acid; and an acidic amino acid, such as glutamic acid and aspartic acid. Examples of the inorganic acid include hydrochloric acid, sulfuric acid, and phosphoric acid. Among these, an organic acid is preferred, and one or more kinds selected from the group consisting of a dicarboxylic acid, a hydroxycarboxylic acid, and an acidic amino acid is more preferred. The dicarboxylic acid is more preferably one or more kinds selected from the group consisting of maleic acid and succinic acid. The hydroxycarboxylic acid is more preferably one or more kinds selected from the group consisting of glycolic acid, lactic acid, and malic acid. The acidic amino acid is more preferably glutamic acid.

Examples of the alkyldimethylamine and a salt thereof (v) include N,N-dimethylbehenylamine, N,N-dimethylstearylamine, and organic acid salts thereof, and N,N-dimethylbehenylamine lactate and N,N-dimethylstearylamine glycolate are preferred.

Examples of the alkoxyalkyldimethylamine and a salt thereof (vi) include N,N-dimethyl-3-hexadecyloxypropylamine, N,N-dimetyl-3-octadecyloxypropylamine, and organic acid salts thereof, and N,N-dimethyl-3-hexadecyloxypropylamine or a salt thereof and N,N-dimetyl-3-octadecyloxypropylamine (stearoxypropyldimethylamine) or a salt thereof are preferred.

Examples of the alkylamidoalkyldimethylamine and a salt thereof (vii) include N-[3-(dimethylamino)propyl]docosanamide, N-[3-(dimethylamino)propyl]stearamide, and organic acid salts thereof, and N-[3-(dimethylamino)propyl]docosanamide lactate and N-[3-(dimethylamino)propyl]stearamide glycolate are preferred.

One kind or two or more kinds of the component (A) may be used.

Among the above, the component (A) is preferably one or more kinds selected from the group consisting of the alkyltrimethylammonium salt (i), the
alkoxyalkyltrimethylammonium salt (ii), the dialkyldimethylammonium salt (iii), the alkylamidoalkyltrimethylammonium salt (iv), the alkyldimethylamine and a salt thereof (v), the alkoxyalkyldimethylamine and a salt thereof (vi), and the alkylamidoalkyldimethylamine and a salt thereof (vii), and more preferably one or more kinds selected from the group consisting of the alkyltrimethylammonium salt (i) and the
alkylamidoalkyltrimethylammonium salt (iv), in the viewpoints of the enhancements of the low temperature stability, the quality of foam for the capability of protecting hair during application, and the smoothness imparted to the hair and the suppression of entanglement of the hair during time of rinsing.

The content or the blending amount of the component (A) in the hair cosmetic composition is preferably 0.08% by mass or more, more preferably 0.10% by mass or more, and further preferably 0.50% by mass or more, and is preferably 5% by mass or less, more preferably 4% by mass or less, and further preferably 3% by mass or less, in the viewpoints of the enhancements of the low temperature stability, the quality of foam for the capability of protecting hair during application, and the smoothness imparted to the hair and the suppression of entanglement of the hair during time of rinsing. More specifically, the content or the blending amount of the component (A) in the hair cosmetic composition is preferably 0.08 to 5% by mass, more preferably 0.08 to 4% by mass, further preferably 0.08 to 3% by mass, still further preferably 0.10 to 3% by mass, and still more further preferably 0.50 to 3% by mass.

### <Component (B): Amphoteric Surfactant>

The hair cosmetic composition contains or is prepared by blending an amphoteric surfactant as the component (B) (which may be hereinafter referred simply to as "(B)").

Examples of the amphoteric surfactant include a betaine type surfactant, an amine oxide type surfactant, and an amino acid type surfactant. Among these, the betaine type surfactant is preferred in the viewpoints of the enhancements of the low temperature stability, the quality of foam for the capability of protecting hair during application, and the smoothness imparted to the hair and the suppression of entanglement of the hair during time of rinsing.

Examples of the betaine type surfactant include a carboxy betaine type, such as an alkyldimethylaminoacetic acid betaine and a fatty acid amidopropyl betaine; a sulfo betaine type, such as an alkylsulfo betaine and an alkylhydroxysulfo betaine; an imidazoline-based betaine type; and a phospho betaine type.

Preferred examples of the alkyldimethylaminoacetic acid betaine include compounds having an acyl group having 8 or more and 22 or less, more preferably 10 or more and 20 or less, carbon atoms, and specific examples thereof include lauryldimethylaminoacetic acid betaine and stearyldimethylaminoacetic acid betaine.

Preferred examples of the fatty acid amidopropyl betaine include compounds having an acyl group having 8 or more and 18 or less, more preferably 10 or more and 16 or less, carbon atoms, and specific examples thereof include lauric acid amidopropyl betaine (lauramidopropyl betaine), palm kernel oil fatty acid amidopropyl betaine, and coconut oil fatty acid amidopropyl betaine (cocamidopropyl betaine).

Preferred examples of the alkylsulfo betaine include an alkylsulfo betaine having an alkyl group having 8 or more and 22 or less, more preferably 10 or more and 18 or less, carbon atoms. Specific examples of the alkylsulfo betaine include lauryldimethylsulfoethyl betaine, lauryldimethylsulfopropyl betaine, myristyldimethylsulfoethyl betaine, myristyldimethylsulfopropyl betaine, stearyldimethylsulfoethyl betaine, stearyldimethylsulfopropyl betaine, and coconut oil fatty acid dimethylsulfopropyl betaine.

Preferred examples of the alkylhydroxysulfo betaine include an alkylhydroxysulfo betaine having an alkyl group having 8 or more and 22 or less, more preferably 10 or more and 18 or less, carbon atoms and at least one hydroxy group. Specific examples of the alkylhydroxysulfo betaine include lauryldimethylsulfo(hydroxyethyl) betaine, lauryldimethylsulfo(hydroxypropyl) betaine (lauryl hydroxysultine), myristyldimethylsulfo(hydroxyethyl) betaine, myristyldimethylsulfo(hydroxypropyl) betaine, stearyldimethylsulfo(hydroxypropyl) betaine, bis(2-hydroxyethyl)sulfoethylbetaine, and laurylbis(2-hydroxyethyl)sulfopropyl betaine. Among these, lauryldimethylsulfo(hydroxypropyl) betaine (lauryl hydroxysultine) represented by the following formula is preferred.

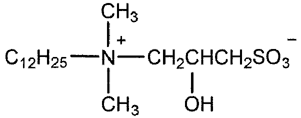

Examples of the imidazoline-based betaine type include a 2-alkyl-N-carboxymethyl-N-hydroxyethylimidazolinium betaine, and specific examples thereof include sodium cocoamphoacetate.

Examples of the phosphobetaine type include laurylhydroxyphospho betaine.

One kind or two or more kinds of the component (B) may be used.

Among the above, the component (B) is more preferably one or more kinds selected from the group consisting of the carboxybetaine type, the sulfobetaine type, and the imidazoline type, further preferably one or more kinds selected from the group consisting of the alkyldimethylaminoacetic acid betaine, the fatty acid amidopropyl betaine, the alkyl sulfobetaine, the alkylhydroxysulfo betaine, and the 2-alkyl-N-carboxymethyl-N-hydroxyethylimidazolinium betaine, and still further preferably one or more kinds selected from the group consisting of the fatty acid amidopropyl betaine, the alkylhydroxysulfo betaine, and the 2-alkyl-N-carboxymethyl-N-hydroxyethylimidazolinium betaine, in the viewpoints of the enhancements of the low temperature stability, the enhancement of the foam quality, the enhancement of the protection capability for the hair in application, and the smoothness imparted to the hair and the suppression of entanglement of the hair during time of rinsing.

The content or the blending amount of the component (B) in the hair cosmetic composition is preferably 0.08% by mass or more, more preferably 0.10% by mass or more, further preferably 0.50% by mass or more, and still further preferably 1% by mass or more, and is preferably 15% by mass or less, more preferably 12% by mass or less, further preferably 10% by mass or less, still further preferably 8% by mass or less, and still more further preferably 5% by mass or less, in the viewpoints of the enhancements of the low temperature stability, the enhancement of the foam quality, the enhancement of the protection capability for the hair in application, and the smoothness imparted to the hair and the suppression of entanglement of the hair during time of rinsing. More specifically, the content or the blending amount of the component (B) in the hair cosmetic composition is preferably 0.08 to 15% by mass, more preferably 0.08 to 12% by mass, further preferably 0.08 to 10% by mass, still further preferably 0.10 to 10% by mass, still more further preferably 0.50 to 8% by mass, yet further preferably 0.50 to 5% by mass, and yet still further preferably 1 to 5% by mass.

The mass ratio [(B)/(A)] of the content or the blending amount of the component (B) to the content or the blending amount of the component (A) in the hair cosmetic composition is 0.4 or more, more preferably 0.7 or more, further preferably 1.0 or more, still further preferably 1.5 or more, and still more further preferably 1.7 or more, and is 5 or less, preferably 4.5 or less, more preferably 4.0 or less, further preferably 3.5 or less, and still further preferably 3.0 or less, in the viewpoints of the enhancements of the low temperature stability, the quality of foam for the capability of protecting hair during application, and the smoothness imparted to the hair and the suppression of entanglement of the hair during time of rinsing. More specifically, the range of the mass ratio [(B)/(A)] is preferably 0.7 to 4.5, more preferably 1.0 to 4.0, further preferably 1.5 to 3.5, and still further preferably 1.7 to 3.0.

### <Component (C)>

The hair cosmetic composition contains or is prepared by blending one or more kinds selected from the group consisting of a nonionic surfactant (C1), a modified silicone having a cationic group (C2), and a sugar alcohol having 4 or more carbon atoms (C3) as the component (C). The use of the component (C) in addition to the component (A) and the component (B) can enhance the capability to protect the hair during application, and imparts favorable smoothness to the hair and suppression of the entanglement of the hair during time of rinsing.

### [Component (C1): Nonionic Surfactant]

Examples of the nonionic surfactant as the component (C1) (which may be hereinafter referred simply to as a "component (C1)" or "(C1)") include a polyoxyalkylene alkyl ether, a polyoxyalkylene alkenyl ether, a polyoxyalkylene sorbitan fatty acid ester, a polyoxyalkylene fatty acid ester, an alkyl glucoside, an alkyl alkanolamide, an alkyl glyceryl ether, a higher fatty acid sucrose ester, a polyglycerin fatty acid ester, a polyoxyethylene hydrogenated castor oil, and an alkylsaccharide.

Among the above, the component (C1) is preferably one or more kinds selected from the group consisting of the polyoxyalkylene alkyl ether, the polyoxyalkylene alkenyl ether, the polyoxyalkylene sorbitan fatty acid ester, the polyoxyalkylene fatty acid ester, the alkyl glucoside, the alkyl alkanolamide, and the alkyl glyceryl ether, and more preferably one or more kinds selected from the group consisting of the polyoxyalkylene alkyl ether, the alkyl glucoside, the alkyl alkanolamide, and the alkyl glyceryl ether, in the viewpoints of the enhancements of the protection capability for the hair in application, and the smoothness imparted to the hair and the suppression of entanglement of the hair during time of rinsing.

The alkyl group in the polyoxyalkylene alkyl ether, the alkyl glucoside, the alkyl alkanolamide, the alkyl glyceryl ether, and the alkylsaccharide, the alkenyl group in the polyoxyalkylene alkenyl ether, and the fatty acid in the polyoxyalkylene sorbitan fatty acid ester, polyoxyalkylene fatty acid ester, the higher fatty acid sucrose esters, and the polyglycerin fatty acid ester each preferably have 8 or more and 22 or less carbon atoms, and more preferably have 8 or more and 18 or less carbon atoms.

Preferred examples of the polyoxyalkylene alkyl ether include a polyoxyethylene alkyl ether or a polyoxypropylene alkyl ether having an alkyl group having 8 or more and 22 or less, more preferably 8 or more and 18 or less, further preferably 8 or more and 12 or less, carbon atoms. Specific examples thereof include "Emulgen 103" (laureth-3: PEG-3 lauryl ether), "Emulgen 116" (laureth-16: PEG-16 lauryl ether), "Emulgen 306P" (steareth-6: polyoxyethylene(6) stearyl ether), and "Kao Sofcare GP-1" (PPG-3 caprylyl ether), all produced by Kao Corporation.

Preferred examples of the alkyl glucoside include an alkyl glucoside having an alkyl group having 8 or more and 22 or less, more preferably 8 or more and 18 or less, further preferably 8 or more and 12 or less, carbon atoms, and specific examples thereof include "Mydol 10" (decyl glucoside) and "AG-124" (lauryl glucoside), all produced by Kao Corporation.

Preferred examples of the alkyl alkanolamide include a fatty acid ethanolamide or a fatty acid diethanolamide having an acyl group having 8 or more and 22 or less, more preferably 8 or more and 18 or less, further preferably 8 or more and 12 or less, carbon atoms, and specific examples thereof include "Aminone PK-02S" (palm kernel oil fatty acid diethanolamide), "Aminone L-02" (lauric acid diethanolamide), and "Aminone C-11S" (coconut oil fatty acid N-methylethanolamide), all produced by Kao Corporation.

Preferred examples of the alkyl glyceryl ether include an alkyl glyceryl ether having an alkyl group having 8 or more and 22 or less, more preferably 8 or more and 18 or less, further preferably 8 or more and 12 or less, carbon atoms, and specific examples thereof include "Penetol GE-ID" (isodecyl glyceryl ether) produced by Kao Corporation.

One kind or two or more kinds of the component (C1) may be used.

The content or the blending amount of the component (C1) in the hair cosmetic composition is preferably 0.08% by mass or more, more preferably 0.10% by mass or more, and further preferably 0.30% by mass or more, and is preferably 6% by mass or less, more preferably 4% by mass or less, and further preferably 2% by mass or less, in the viewpoints of the enhancements of the protection capability for the hair in application, and the smoothness imparted to the hair and the suppression of entanglement of the hair during time of rinsing. More specifically, the content or the blending amount of the component (C1) in the hair cosmetic composition is preferably 0.08 to 6% by mass, more preferably 0.10 to 4% by mass, and further preferably 0.30 to 2% by mass.

The mass ratio (((A)+(B))/(C1)) of the total content or the total blending amount of the component (A) and the component (B) to the content or the blending amount of the component (C1) in the hair cosmetic composition (which may be hereinafter referred simply to as a "mass ratio (((A)+(B))/(C1))") is preferably 0.1 or more, more preferably 0.5 or more, further preferably 1 or more, and still further preferably 3 or more, and is preferably 25 or less, more preferably 20 or less, further preferably 15 or less, and still further preferably 10 or less, in the viewpoints of the enhancements of the protection capability for the hair in application, and the smoothness imparted to the hair and the suppression of entanglement of the hair during time of rinsing. More specifically, the range of the mass ratio (((A)+(B))/(C1)) is preferably 0.1 to 25, more preferably 0.5 to 20, further preferably 1 to 15, and still further preferably 3 to 10.

### [Component (C2): Modified Silicone having Cationic Group]

The modified silicone having a cationic group as the component (C2) (which may be hereinafter referred simply to as a "component (C2)" or "(C2)") is a modified silicone having a cationic group introduced thereto, and is preferably a modified silicone having a cationic group on a side chain or at an end of the polysiloxane skeleton.

The cationic group means a cation group or a group capable of becoming a cation group through ionization. Specific examples thereof include a primary amino group, a secondary amino group, a tertiary amino group, and a quaternary ammonium group.

The component (C2) has a nitrogen content that is preferably 0.1% by mass or more, more preferably 0.2% by mass or more, further preferably 0.5% by mass or more, and still further preferably 0.8% by mass or more, and is preferably 2.5% by mass or less, more preferably 2.0% by mass or less, further preferably 1.8% by mass or less, and still further preferably 1.5% by mass or less, in the viewpoints of the enhancements of the protection capability for the hair in application, and the smoothness imparted to the hair and the suppression of entanglement of the hair during time of rinsing. More specifically, the range of the nitrogen content of the component (C2) is preferably 0.1 to 2.5% by mass, more preferably 0.2 to 2.0% by mass, further preferably 0.5 to 1.8% by mass, and still further preferably 0.8 to 1.5% by mass. The nitrogen content of the component (C2) is a value that is measured according to the potentiometric titration method defined in JIS K0113:2005.

The component (C2) is preferably one or more kinds selected from the group consisting of an amino polyether-modified silicone and a quaternary ammonium cation-modified silicone in the viewpoints of the enhancements of the protection capability for the hair in application, and the smoothness imparted to the hair and the suppression of entanglement of the hair during time of rinsing.

The amino polyether-modified silicone is more specifically preferably an amino polyether-modified silicone having a structure represented by the following general formula (1-1): 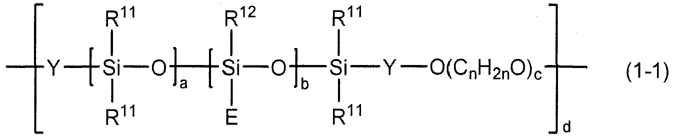 wherein in the formula (1-1), R¹¹ represents a monovalent hydrocarbon group having 1 or more and 6 or less carbon atoms; R¹² represents R¹¹ or E; E represents a monovalent group represented by -R¹³-Z (wherein R¹³ represents a single bond or a divalent hydrocarbon group having 1 or more and 20 or less carbon atoms, and Z represents a primary to tertiary amino group-containing group); Y represents an alkylene group having 1 or more and 6 or less carbon atoms; a represents a number of 2 or more; b represents a number of 1 or more; c represents a number of 4 or more and 100 or less; d represents a number of 1 or more; and n represents a number of 2 or more and 10 or less, provided that the structural units in brackets are not restricted in bonding order and may have any of a block bonding mode and a random bonding mode, the c number of groups represented by CₙH₂ₙO may be the same as or different from each other, and the plural groups represented by each of R¹¹, R¹², and E may be the same as or different from each other.

In the general formula (1-1), plural groups represented by R¹¹ each are independently preferably an alkyl group having 1 or more and 6 or less carbon atoms or a phenyl group, more preferably a methyl group or an ethyl group, and further preferably a methyl group.

In the general formula (1-1), R¹² represents R¹¹ or E; E represents a monovalent group represented by -R¹³-Z (wherein R¹³ represents a single bond or a divalent hydrocarbon group having 1 or more and 20 or less carbon atoms). R¹³ preferably represents a divalent hydrocarbon group having 1 or more and 20 or less carbon atoms, more preferably an alkylene group having 1 or more and 20 or less carbon atoms, further preferably a linear or branched alkylene group having 1 or more and 6 or less carbon atoms, still further preferably a methylene group, an ethylene group, a trimethylene group, a propylene group, a tetramethylene group, or a hexamethylene group, and still more further preferably a trimethylene group or a propylene group.

Z represents a primary to tertiary amino group-containing group, and preferably represents an amino group-containing group represented by -N(R¹⁴)₂, -NR¹⁴(CH₂)ₘN(R¹⁴)₂, or -NR¹⁴(CH₂)ₘN(R)CO-R¹⁶. R¹⁴ and R¹⁵ each independently represent a hydrogen atom or an alkyl group having 1 or more and 3 or less carbon atoms, and preferably a hydrogen atom or a methyl group. R¹⁶ represents an alkyl group having 1 or more and 3 or less carbon atoms. m represents a number of 1 or more and 6 or less, and preferably a number of 2 or more and 4 or less.

In the general formula (1-1), E preferably represents -(CH₂)₃-NH₂, -(CH₂)₃-N(CH₃)₂, -(CH₂)₃-NH-(CH₂)₂-NH₂, or -(CH₂)₂-NH-(CH₂)₂-N(CH₃)₂, and more preferably -(CH₂)₃-NH-(CH₂)₂-NH₂.

In the general formula (1-1), Y preferably represents an ethylene group, a propylene group, a trimethylene group, a n-butylene group (tetramethylene group), or an *i*-butylene group, and more preferably a n-butylene group or an i-butylene group. The i-butylene group referred herein encompasses -CH(CH₃)CH₂CH₂-, -CH2CH(CH3)CH2-, and - CH₂CH₂CH(CH₃)-.

In the general formula (1-1), a represents a number of 2 or more, b represents a number of 1 or more, c represents a number of 4 or more and 100 or less, and d represents a number of 1 or more. a preferably represents a number of 2 or more and 1,000 or less, and more preferably a number of 2 or more and 100 or less. b preferably represents a number of 1 or more and 50 or less, c preferably represents a number of 4 or more and 50 or less, and more preferably a number of 10 or more and 18 or less, and d preferably represents a number of 2 or more and 100 or less.

In the general formula (1-1), n represents a number of 2 or more and 10 or less, preferably a number of 2 or more and 6 or less, and more preferably a number of 2 or more and 4 or less.

The amino polyether-modified silicone is preferably an amino polyether-modified silicone having a structure represented by the following general formula (1-2): 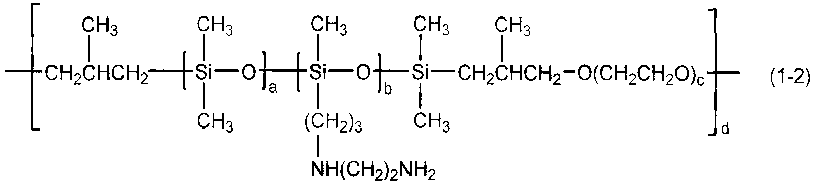 wherein in the formula (1-2), a to d are the same as above.

The amino polyether-modified silicone may be used alone or as a combination of two or more kinds thereof.

The amino polyether-modified silicone used may be a commercially available amino polyether-modified silicone. Examples of the amino polyether-modified silicone having a repeating unit represented by the general formula (1-2) include "Silstyle 104" ((bisisobutyl PEG-14/amodimethicone) copolymer, nitrogen content: 1.2% by mass), "Dowsil SS-3588" ((bisisobutyl PEG-15/amodimethicone) copolymer, nitrogen content: 1.0% by mass), "Silstyle 401" ((bisisobutyl PEG/PPG-20/35/amodimethicone) copolymer, nitrogen content: 0.2% by mass), and "Silstyle 201" ((bisisobutyl PEG-14/amodimethicone) copolymer, nitrogen content: 1.2% by mass), all produced by Dow Toray Co., Ltd.

The quaternary ammonium cation-modified silicone is more specifically preferably a side chain type quaternary ammonium cation-modified silicone having a structure represented by the following general formula (2-1) or an end type quaternary ammonium cation-modified silicone having a structure represented by the following general formula (2-2): 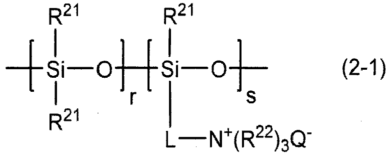 wherein in the formula (2-1), R²¹ represents a monovalent hydrocarbon group having 1 or more and 6 or less carbon atoms; R²² represents a hydrogen atom, a hydrocarbon group having 1 or more and 20 or less carbon atoms, or a hydrocarbon group containing an amide bond having 1 or more and 20 or less carbon atoms; L represents a divalent organic group; Q⁻represents a counter ion of the quaternary ammonium ion; r represents a number of 2 or more; and s represents a number of 1 or more, provided that the structural units in brackets are not restricted in bonding order and may have any of a block bonding mode and a random bonding mode, and the plural groups represented by each of R²¹ and R²² may be the same as or different from each other, 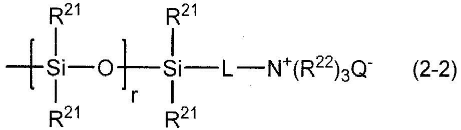 wherein in the formula (2-2), R²¹, R²², L, Q⁻, and r are the same as above.

In the general formulae (2-1) and (2-2), plural groups represented by R²¹ each are independently preferably an alkyl group having 1 or more and 6 or less carbon atoms or a phenyl group, more preferably a methyl group or an ethyl group, and further preferably a methyl group.

In the general formulae (2-1) and (2-2), R²² preferably represents a hydrocarbon group having 1 or more and 20 or less carbon atoms or a hydrocarbon group containing an amide bond having 1 or more and 20 or less carbon atoms, and at least one of the plural groups represented by R²² is preferably a hydrocarbon group containing an amide bond having 1 or more and 20 or less carbon atoms.

In the general formulae (2-1) and (2-2), L preferably represents ^{∗1}-R²³-CH₂-CHOH-CH₂-^{∗2}, wherein R²³ represents a divalent organic group, and preferably an alkylene group having 1 or more and 20 or less carbon atoms or an oxyalkylene group having 1 or more and 20 or less carbon atoms, ^{∗1} represents a bonding site to the silicon atom, and ^{∗2} represents a bonding site to the nitrogen atom.

In the general formula (2-1), r preferably represents a number of 2 or more and 200 or less, and preferably a number of 2 or more and 50 or less, and s represents a number of 1 or more and 50 or less, more preferably a number of 2 or more and 20 or less, and further preferably a number of 2 or more and 10 or less.

In the general formula (2-2), r preferably represents a number of 2 or more and 200 or less, and more preferably a number of 2 or more and 100 or less.

In the general formulae (2-1) and (2-2), Q⁻ represents an anion, for example, a halide ion, such as a chloride ion and a bromide ion; and an organic acid ion, such as an alkylsulfate ion having 1 or more and 3 or less carbon atoms, an acetate ion, a lactate ion, a benzoate ion, an adipate ion, a formate ion, a malate ion, and a glycolate ion. Among these, a lactate ion is preferred.

The end type quaternary ammonium cation-modified silicone is more preferably a quaternary ammonium cation-modified silicone having a structure represented by the following general formula (2-3): 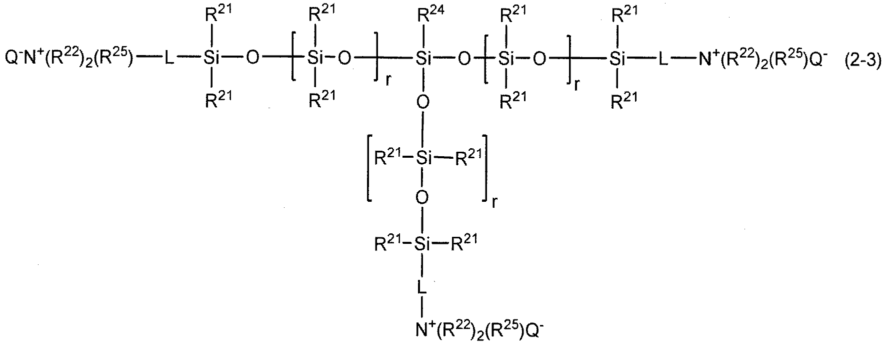 wherein in the general formula (2-3), R²¹, R²², L, Q⁻, and r are the same as above; R²⁴ represents an alkyl group having 1 or more and 6 or less carbon atoms or a phenyl group; and R²⁵ represents a hydrocarbon group having 1 or more and 20 or less carbon atoms or a hydrocarbon group containing an amide bond having 1 or more and 20 or less carbon atoms.

In the general formula (2-3), plural groups represented by R²¹ each are independently preferably an alkyl group having 1 or more and 6 or less carbon atoms or a phenyl group, more preferably a methyl group or an ethyl group, and further preferably a methyl group.

In the general formula (2-3), R²² preferably represents a hydrocarbon group having 1 or more and 20 or less carbon atoms, and more preferably a methyl group.

In the general formula (2-3), R²⁴ preferably represents an alkyl group having 1 or more and 6 or less carbon atoms or a phenyl group, and more preferably a phenyl group.

In the general formula (2-3), Q⁻ preferably represents a lactate ion, and r preferably represents a number of 2 or more and 200 or less, and more preferably a number of 2 or more and 100 or less.

In the general formula (2-3), R²⁵ preferably represents a hydrocarbon group having 1 or more and 20 or less carbon atoms or a hydrocarbon group containing an amide bond having 1 or more and 20 or less carbon atoms, more preferably a hydrocarbon group containing an amide bond having 1 or more and 20 or less carbon atoms, and further preferably -R²⁶-NHCO-R²⁷.

Herein, R²⁶ represents an alkylene group having 1 or more and 18 or less carbon atoms or an oxyalkylene group, and preferably an alkylene group having 1 or more and 18 or less carbon atoms, and R²⁷ represents an alkyl group having 1 or more and 18 or less carbon atoms.

In the general formula (2-3), L preferably represents ^{∗1}-R²³-CH₂-CHOH-CH2-^{∗2}, wherein R²³ preferably represents an alkylene group having 1 or more and 20 or less carbon atoms or an oxyalkylene group having 1 or more and 20 or less carbon atoms, and more preferably an oxyalkylene group having 1 or more and 20 or less carbon atoms, and ^{∗1} and ^{∗2} are the same as above.

Examples of the quaternary ammonium cation-modified silicone include silicone quaternium-1, silicone quaternium-2, silicone quaternium-3, silicone quaternium-4, silicone quaternium-5, silicone quaternium-6, silicone quaternium-7, silicone quaternium-8, silicone quaternium-9, silicone quaternium-10, silicone quaternium-11, silicone quaternium-12, silicone quaternium-15, silicone quaternium-16, silicone quaternium-17, silicone quaternium-18, silicone quaternium-20, silicone quaternium-21, silicone quaternium-22, quaternium-80, silicone quaternium-2 panthenol succinate, and silicone quaternium-16/glycidyl dimethicone crosspolymer.

The component (C2) preferably contains the amino polyether-modified silicone in the viewpoints of the enhancements of the protection capability for the hair in application, and the smoothness imparted to the hair and the suppression of entanglement of the hair during time of rinsing. In this case, the amino polyether-modified silicone and the quaternary ammonium cation-modified silicone may be used in combination as the component (C2).

In the case where the component (C2) contains the amino polyether-modified silicone, the content of the amino polyether-modified silicone in the component (C2) is preferably 50% by mass or more, more preferably 70% by mass or more, and further preferably 90% by mass or more.

The content or the blending amount of the component (C2) in the hair cosmetic composition is preferably 0.08% by mass or more, more preferably 0.10% by mass or more, and further preferably 0.15% by mass or more, and is preferably 3% by mass or less, more preferably 2% by mass or less, and further preferably 1% by mass or less, in the viewpoints of the enhancements of the protection capability for the hair in application, and the smoothness imparted to the hair and the suppression of entanglement of the hair during time of rinsing. More specifically, the content or the blending amount of the component (C2) in the hair cosmetic composition is preferably 0.08 to 3% by mass, more preferably 0.10 to 2% by mass, and further preferably 0.15 to 1% by mass.

### [Component (C3): Sugar Alcohol having 4 or more Carbon Atoms)

The sugar alcohol as the component (C3) is a sugar alcohol having 4 or more carbon atoms, and examples thereof include erythritol, xylitol, sorbitol, mannitol, maltitol, and lactitol. Among these, one or more kinds selected from the group consisting of erythritol and mannitol is preferred in the viewpoints of the enhancements of the protection capability for the hair in application, and the smoothness imparted to the hair and the suppression of entanglement of the hair during time of rinsing.

The content or the blending amount of the component (C3) in the hair cosmetic composition is preferably 0.05% by mass or more, more preferably 0.10% by mass or more, and further preferably 0.50% by mass or more, and is preferably 3.0% by mass or less, more preferably 2.5% by mass or less, further preferably 2.0% by mass or less, and still further preferably 1.5% by mass or less, in the viewpoints of the enhancements of the protection capability for the hair in application, and the smoothness imparted to the hair and the suppression of entanglement of the hair during time of rinsing. More specifically, the content or the blending amount of the component (C3) in the hair cosmetic composition is preferably 0.05 to 3.0% by mass, more preferably 0.10 to 2.5% by mass, further preferably 0.50 to 2.0% by mass, and still further preferably 0.50 to 1.5% by mass.

In the present invention, the component (C) is not particularly limited as far as one or more kinds selected from the group consisting of the nonionic surfactant (C1), the modified silicone having a cationic group (C2), and the sugar alcohol having 4 or more carbon atoms (C3) is contained, and an embodiment in which the component (C) contains the nonionic surfactant (C1) (which may be hereinafter referred to as a "first embodiment") and an embodiment in which the component (C) contains one or more kinds selected from the group consisting of the modified silicone having a cationic group (C2) and the sugar alcohol having 4 or more carbon atoms (C3) (which may be hereinafter referred to as a "second embodiment") are preferred in the viewpoints of the enhancements of the low temperature stability, the enhancement of the foam quality, the enhancement of the protection capability for the hair in application, and the smoothness imparted to the hair and the suppression of entanglement of the hair during time of rinsing.

Accordingly, the first embodiment of the present invention relates to a hair cosmetic composition
containing or being prepared by blending a component (A), a component (B), and a component (C) below:
component (A): a cationic surfactant,
component (B): an amphoteric surfactant, and
component (C): containing a nonionic surfactant (C1),
having a mass ratio [(B)/(A)] of a content or a blending amount of the component (B) to a content or a blending amount of the component (A) of 0.4 or more and 5 or less,
having a content or a blending amount of a higher alcohol of 0.1% by mass or less, and
being used by filling up a non-gas type foam discharge container.

In the first embodiment, the content or the blending amount of the component (C1) in the hair cosmetic composition is as described above.

In the first embodiment, the mass ratio (((A)+(B))/(C1)) of the total content or the total blending amount of the component (A) and the component (B) to the content or the blending amount of the component (C1) in the hair cosmetic composition is preferably 0.1 or more, more preferably 0.5 or more, further preferably 1 or more, and still further preferably 3 or more, and is preferably 25 or less, more preferably 20 or less, further preferably 15 or less, and still further preferably 10 or less, in the viewpoints of the enhancements of the low temperature stability, the enhancement of the protection capability for the hair in application, and the smoothness imparted to the hair and the suppression of entanglement of the hair during time of rinsing. More specifically, the range of the mass ratio (((A)+(B))/(C1)) is preferably 0.1 to 25, more preferably 0.5 to 20, further preferably 1 to 15, and still further preferably 3 to 10.

In the first embodiment, the component (C) preferably contains the component (C2) in addition to the component (C1) in the viewpoints of the enhancements of the protection capability for the hair in application, and the smoothness imparted to the hair and the suppression of entanglement of the hair during time of rinsing.

In the case where the component (C) contains the component (C1) and the component (C2), the mass ratio [(C2)/(C1)] of the content or the blending amount of the component (C2) to the content or the blending amount of the component (C1) in the hair cosmetic composition (which may be hereinafter referred simply to as a "mass ratio [(C2)/(C1)]") is preferably 0.08 or more, more preferably 0.1 or more, and further preferably 0.2 or more, and is preferably 10 or less, more preferably 5 or less, further preferably 3 or less, and still further preferably 2 or less, in the viewpoints of the enhancements of the protection capability for the hair in application, and the smoothness imparted to the hair and the suppression of entanglement of the hair during time of rinsing. More specifically, the mass ratio [(C2)/(C1)] is preferably 0.08 to 10, more preferably 0.1 to 5, further preferably 0.1 to 3, and still further preferably 0.2 to 2.

The second embodiment of the present invention relates to a hair cosmetic composition
containing or being prepared by blending a component (A), a component (B), and a component (C) below:
component (A): a cationic surfactant,
component (B): an amphoteric surfactant, and
component (C): containing one or more kinds selected from the group consisting of a modified silicone having a cationic group (C2) and a sugar alcohol having 4 or more carbon atoms (C3),
having a mass ratio [(B)/(A)] of a content or a blending amount of the component (B) to a content or a blending amount of the component (A) of 0.4 or more and 5 or less,
having a content or a blending amount of a higher alcohol of 0.1% by mass or less, and
being used by filling up a non-gas type foam discharge container.

In the second embodiment, the total content or the total blending amount of the component (C2) and the component (C3) in the hair cosmetic composition is preferably 0.1% by mass or more, more preferably 0.3% by mass or more, and further preferably 0.5% by mass or more, and is preferably 5% by mass or less, more preferably 3% by mass or less, and further preferably 2% by mass or less, in the viewpoints of the enhancements of the low temperature stability, the enhancement of the protection capability for the hair in application, and the smoothness imparted to the hair and the suppression of entanglement of the hair during time of rinsing. More specifically, the total content or the total blending amount of the component (C2) and the component (C3) in the hair cosmetic composition is preferably 0.1 to 5% by mass, more preferably 0.3 to 3% by mass, and further preferably 0.5 to 2% by mass.

In the second embodiment, the mass ratio (((A)+(B))/((C2)+(C3))) of the total content or the total blending amount of the component (A) and the component (B) to the total content or the total blending amount of the component (C2) and the component (C3) in the hair cosmetic composition (which may be hereinafter referred simply to as a "mass ratio (((A)+(B))/((C2)+(C3)))") is preferably 0.1 or more, more preferably 0.3 or more, and further preferably 1 or more, and is preferably 40 or less, more preferably 35 or less, further preferably 30 or less, still further preferably 20 or less, and still more further preferably 15 or less, in the viewpoints of the enhancements of the low temperature stability, the enhancement of the protection capability for the hair in application, and the smoothness imparted to the hair and the suppression of entanglement of the hair during time of rinsing. More specifically, the range of the mass ratio (((A)+(B))/((C2)+(C3))) is preferably 0.1 to 40, more preferably 0.3 to 35, further preferably 1 to 30, still further preferably 1 to 20, and still more further preferably 1 to 15.

In the second embodiment, the component (C2) and the component (C3) may be used in combination as the component (C) in the viewpoints of the enhancements of the low temperature stability, the enhancement of the protection capability for the hair in application, and the smoothness imparted to the hair and the suppression of entanglement of the hair during time of rinsing.

Specifically, in the case where the component (C) contains the component (C2) and the component (C3), the mass ratio ((C3)/(C2)) of the content or the blending amount of the component (C3) to the content or the blending amount of the component (C2) in the hair cosmetic composition (which may be hereinafter referred simply to as a "mass ratio ((C3)/(C2))") is preferably 0.08 or more, more preferably 0.5 or more, and further preferably 1 or more, and is preferably 10 or less, more preferably 5 or less, and further preferably 3 or less, in the viewpoints of the enhancements of the low temperature stability, the enhancement of the protection capability for the hair in application, and the smoothness imparted to the hair and the suppression of entanglement of the hair during time of rinsing. More specifically, the mass ratio ((C3)/(C2)) is preferably 0.08 to 10, more preferably 0.5 to 5, and further preferably 1 to 3.

The total content or the total blending amount of the component (A), the component (B), and the component (C) in the hair cosmetic composition is preferably 0.2% by mass or more, more preferably 0.3% by mass or more, further preferably 0.5% by mass or more, and still further preferably 1% by mass or more, and is preferably 20% by mass or less, more preferably 15% by mass or less, and further preferably 10% by mass or less. More specifically the total content or the total blending amount of the component (A), the component (B), and the component (C) in the hair cosmetic composition is preferably 0.2 to 20% by mass, more preferably 0.3 to 15% by mass, further preferably 0.5 to 10% by mass, and still further preferably 1 to 10% by mass.

### <Higher Alcohol>

In the hair cosmetic composition of the present invention, the content or the blending amount of a higher alcohol is 0.1% by mass or less, and thereby the low temperature stability and the foam quality are enhanced.

From the above-mentioned viewpoints, the content or the blending amount of a higher alcohol in the hair cosmetic composition is preferably 0.06% by mass or less, more preferably 0.03% by mass or less, and further preferably 0% by mass.

The higher alcohol preferably has 12 or more and 22 or less carbon atoms. Examples of the higher alcohol include cetyl alcohol, oleyl alcohol, stearyl alcohol, isostearyl alcohol, 2-octyldodecanol, myristyl alcohol, behenyl alcohol, and cetostearyl alcohol. One kind or two or more kinds of the higher alcohols may be contained in such an amount that satisfies the aforementioned range of the content or blending amount.

In the case where two or more kinds of the higher alcohols are used in combination, the content or the blending amount of the higher alcohol in the hair cosmetic composition is the total content or the total blending amount of the higher alcohols.

### <Component (D): Anionic Surfactant>

The hair cosmetic composition may further contain or may be prepared by further blending an anionic surfactant as a component (D) (which may be hereinafter referred simply to as a "component (D)" or "(D)").

Examples of the component (D) include an alkylbenzene sulfonate, an alkyl or alkenyl ether sulfate, an alkyl or alkenyl sulfate, an alkyl sulfonate, a saturated or unsaturated fatty acid salt, an alkyl or alkenyl ether carboxylate, an α-sulfo fatty acid salt, an N-acylamino acid, a phosphoric acid mono- or diester, and a sulfosuccinate ester, and one kind or two or more kinds thereof may be used.

Examples of the counter ion of the anionic group of the component (D) include an alkali metal ion, such as a sodium ion and a potassium ion; an alkaline earth metal ion, such as a calcium ion and a magnesium ion; an ammonium ion; and an alkanol ammonium having 1 to 3 alkanol groups having 2 or 3 carbon atoms (such as monoethanolammonium, diethanolammonium, triethanolammonium, and triisopropanolammonium).

The component (D) is preferably one or more kinds selected from the group consisting of an alkyl sulfate, an alkyl ether sulfate, and an alkyl ether carboxylate. Examples of the alkyl ether sulfate include a polyoxyethylene alkyl ether sulfate, such as sodium laureth sulfate, and examples of the alkyl ether carboxylate include a polyoxyethylene alkyl ether acetate, such as sodium laureth acetate.

The component (D) is more preferably one or more kinds selected from the group consisting of an alkyl ether sulfate and an alkyl ether carboxylate, and further preferably one or more kinds selected from the group consisting of a polyoxyethylene alkyl ether sulfate and a polyoxyethylene alkyl ether acetate.

In the case where the hair cosmetic composition contains or is prepared by blending the component (D), the content or the blending amount of the component (D) in the hair cosmetic composition is preferably 0.005% by mass or more, more preferably 0.01% by mass or more, and further preferably 0.02% by mass or more, and is preferably 3% by mass or less, more preferably 1% by mass or less, further preferably 0.5% by mass or less, and still further preferably 0.1% by mass or less. More specifically, the content or the blending amount of the component (D) in the hair cosmetic composition is preferably 0.005 to 3% by mass, more preferably 0.01 to 1% by mass, further preferably 0.01 to 0.5% by mass, still further preferably 0.01 to 0.1% by mass, and still more further preferably 0.02 to 0.1% by mass.

### <Cationic Polymer>

The hair cosmetic composition may further contain or may be prepared by further blending a cationic polymer in the viewpoints of the enhancements of the protection capability for the hair in application, and the smoothness imparted to the hair and the suppression of entanglement of the hair during time of rinsing.

In the present invention, the "cationic polymer" means a water-soluble polymer having a cationic group and having overall cationic charge, and the cationic group is as described above.

Examples of the cationic polymer include a cationized polygalactomannan, such as cationized guar gum, cationized tara gum, and cationized locust bean gum; a cationized cellulose; a cationized hydroxyalkyl cellulose, such as cationized hydroxyethyl cellulose and cationized hydroxypropyl cellulose; a cationic starch; a cationized polyvinyl alcohol;
a quaternized dialkylaminoalkyl (meth)acrylate polymer, such as a vinylpyrrolidone/N,N-dimethylaminoethyl methacrylate diethyl sulfate copolymer and an N,N-dimethylaminoethyl methacrylate diethyl sulfate/N,N-dimethylacrylamide/polyethylene glycol dimethacrylate copolymer;
a diallyl quaternized ammonium salt polymer, such as a polydiallyldimethylammonium chloride, a diallyldimethylammonium chloride/acrylic acid copolymer, a diallyldimethylammonium chloride/acrylamide copolymer, and a diallyldimethylammonium chloride/acrylic acid/acrylamide copolymer;
a vinylimidazolium trichloride/vinylpyrrolidone copolymer; a vinylpyrrolidone/alkylaminoalkyl (meth)acrylate copolymer; a vinylpyrrolidone/alkylaminoalkyl (meth)acrylate/vinyl caprolactam copolymer; a vinylpyrrolidone/(meth)acrylamide propyl trimethylammonium chloride copolymer; an alkyl acrylamide/(meth)acrylate/alkylaminoalkyl acrylamide/polyethylene glycol (meth)acrylate copolymer; an adipic acid/dimethylaminohydroxypropyl ethylene triamine copolymer; and
cationic polymers described in JP 53-139734 A and JP 60-36407 A, and one kind or two or more kinds thereof may be used. Among these, one or more kinds selected from the group consisting of a cationized polygalactomannan, a cationized hydroxyalkyl cellulose, a quaternized dialkylaminoalkyl (meth)acrylate polymer, and a diallyl quaternized ammonium salt polymer is preferred, and one or more kinds selected from the group consisting of cationized guar gum, a cationized hydroxyalkyl cellulose, and a quaternized dialkylaminoalkyl (meth)acrylate polymer is more preferred.

The weight average molecular weight of the cationic polymer is preferably 10,000 or more, more preferably 50,000 or more, and further preferably 100,000 or more, and is generally 8,000,000 or less, in the viewpoints of the enhancements of the protection capability for the hair in application, and the smoothness imparted to the hair and the suppression of entanglement of the hair during time of rinsing.

The weight average molecular weight may be measured, for example, by gel permeation chromatography (GPC) under the following condition.
Mobile phase: 50 mM LiBr, (1% CH₃COOH/ethanol)/water = 3/7
Column: TSK gel α-M (two columns connected in series)
Standard substance: polyethylene glycol

The cationic polymer used may be a commercially available cationic polymer. Examples thereof include the following.

### (Cationized guar gum)

JAGUAR Excel (produced by Solvay S.A. (Novecare)), JAGUAR C-14-S (produced by Solvay S.A. (Rhodia)) and the like
   (Cationized tara gum)
CATINAL CTR-100 (produced by Toho Chemical Industry Co., Ltd.) and the like (Cationized locust bean gum)
CATINAL CLB-100 (produced by Toho Chemical Industry Co., Ltd.) and the like (Cationized hydroxyethyl cellulose)
Polyquaternium-10 (o-[2-hydroxy-3-(trimethylammonio)propyl]hydroxyethyl cellulose chloride), such as UCare Polymer JR-400 (produced by The Dow Chemical Company), POIZ C-60H (produced by Kao Corporation), POIZ C-150L (produced by Kao Corporation), and the like
Polyquaternium-67: SoftCAT (produced by The Dow Chemical Company), and the like
   (Cationized hydroxypropyl cellulose)
SOFCARE C-HP2W (produced by Kao Corporation) and the like
   (Cationized polyvinyl alcohol)
GOHSENX K-434 (produced by The Nippon Synthetic Chemical Industry Co., Ltd.), CM318 (produced by Kuraray Co., Ltd.), and the like (Vinylpyrrolidone/N,N-dimethylaminoethyl methacrylate diethyl sulfate copolymer)
Polyquaternium-11: GAFQUAT 734 (produced by ISP Japan Ltd.), GAFQUAT 755N (produced by ISP Japan Ltd.), and the like (N,N-Dimethylaminoethyl methacrylate diethyl sulfate/N,N-dimethylacrylamide/polyethylene glycol dimethacrylate copolymer)
Polyquaternium-52: SOFCARE KG-101 W-E (produced by Kao Corporation) and the like
   (Polydiallyldimethylammonium chloride)
Polyquaternium-6: MERQUAT 100 (produced by Lubrizol Advanced Materials, Inc.) and the like
   (Diallyldimethylammonium chloride/acrylic acid copolymer)
Polyquaternium-22: MERQUAT 280 and MERQUAT 295 (all produced by Lubrizol Advanced Materials, Inc.) and the like
   (Diallyldimethylammonium chloride/acrylamide copolymer)
Polyquaternium-7: MERQUAT 550 (produced by Lubrizol Advanced Materials, Inc.) and the like
   (Diallyldimethylammonium chloride/acrylic acid/acrylamide polymer)
Polyquaternium-39: MERQUAT 3331PR (produced by Lubrizol Advanced Materials, Inc.) and the like

In the case where the hair cosmetic composition contains or is prepared by blending the cationic polymer, the content or the blending amount of the cationic polymer in the hair cosmetic composition is preferably 0.01% by mass or more, more preferably 0.05% by mass or more, and further preferably 0.1% by mass or more, in the viewpoints of the enhancements of the protection capability for the hair in application, and the smoothness imparted to the hair and the suppression of entanglement of the hair during time of rinsing, and is preferably 3% by mass or less, more preferably 1% by mass or less, and further preferably 0.5% by mass or less, in the viewpoint of the ease in blending. More specifically, the range of the content or the blending amount of the cationic polymer in the hair cosmetic composition is preferably 0.01 to 3% by mass, more preferably 0.05 to 1% by mass, and further preferably 0.1 to 0.5% by mass.

### <Organic Acid or Inorganic Acid>

The hair cosmetic composition preferably further contains or is preferably prepared by further blending an organic acid or an inorganic acid in the viewpoint of the dissolution of the components (A), (B), and (C). It is also preferable in the viewpoint of the neutralization of an amine compound for forming a salt, in the case where the amine compound, such as an alkyldimethylamine, an alkoxyalkyldimethylamine, or an alkylamidoalkyldimethylamine, among the cationic surfactants is used as the component (A).

Examples of the organic acid or inorganic acid include the organic acids or inorganic acids as described above, and an organic acid is preferred. The organic acid is preferably one or more kinds selected from the group consisting of a dicarboxylic acid, a hydroxycarboxylic acid, and an acidic amino acid, and a hydroxycarboxylic acid is more preferred. The dicarboxylic acid is more preferably one or more kinds selected from the group consisting of maleic acid and succinic acid. The hydroxycarboxylic acid is more preferably one or more kinds selected from the group consisting of glycolic acid, lactic acid, and malic acid. The acidic amino acid is more preferably glutamic acid.

In the case where the organic or inorganic acid is used, the content or the blending amount of the organic acid or inorganic acid in the hair cosmetic composition is preferably 0.005% by mass or more, more preferably 0.01% by mass or more, and further preferably 0.02% by mass or more, in the viewpoint of the dissolution of the components (A), (B), and (C), and is preferably 10% by mass or less, more preferably 8% by mass or less, further preferably 5% by mass or less, still further preferably 1% by mass or less, and still more further preferably 0.5% by mass or less, in the viewpoint of the stability of the product. More specifically, the range of the content or the blending amount of the organic acid or inorganic acid in the hair cosmetic composition is preferably 0.005 to 10% by mass, more preferably 0.01 to 8% by mass, further preferably 0.02 to 5% by mass, still further preferably 0.02 to 1% by mass, and still more further preferably 0.02 to 0.5% by mass.

In the case where an amine compound, such as an alkyldimethylamine, an alkoxyalkyldimethylamine, or an alkylamidoalkyldimethylamine, as the cationic surfactant as the component (A) is used, the organic acid or inorganic acid may be contained or may be blended in such an amount that is appropriate to neutralize the amine compound for forming a salt.

### <Aqueous Medium>

The hair cosmetic composition generally contains or is prepared by blending an aqueous medium. Examples of the aqueous medium include water and an organic solvent. Examples of the organic solvent include a lower alcohol, such as ethanol and isopropyl alcohol; and a low molecular weight diol or triol having 6 or less carbon atoms, such as 1,3-butylene glycol, glycerin, ethylene glycol, propylene glycol, and dipropylene glycol. Among these, water is preferred.

The content or the blending amount of the aqueous medium in the hair cosmetic composition is preferably 60 to 99% by mass, and more preferably 70 to 99% by mass, in the viewpoints of the enhancements of the foam quality and the dischargeability of foam.

### <Additional Components>

The hair cosmetic composition may contain or may be prepared by blending additional components in such an amount that does not impair the objects of the present invention. Examples of the components include an antioxidant, an oil agent, an antidandruff agent, a vitamin agent, a disinfectant, an anti-inflammatory agent, an antiseptic, a chelating agent, a moisturizer, a pearlescent agent, a ceramide compound, a fragrance, and a UV absorbent, which are components that are ordinarily blended in hair cosmetics.

### <pH>

The pH of the hair cosmetic composition is preferably 3.0 or more, more preferably 3.1 or more, and further preferably 3.2 or more, in terms of pH of a 5% aqueous solution, in the viewpoint of the enhancements of the foam quality, and is preferably 7.0 or less, more preferably 6.0 or less, and further preferably 5.0 or less, in terms of pH of the 5% aqueous solution, in the viewpoints of the smoothness and the prevention of entanglement of the hair. More specifically, the range of the pH of the 5% aqueous solution of the hair cosmetic composition is preferably 3.0 to 7.0, more preferably 3.1 to 6.0, and further preferably 3.2 to 5.0.

The pH is a measurement value at 25°C, and can be specifically measured by the method described in the examples.

### <Viscosity at 30°C>

The viscosity at 30°C of the hair cosmetic composition is preferably 0.8 mPa·s or more, more preferably 1 mPa·s or more, further preferably 2 mPa·s or more, and still further preferably 3 mPa·s or more, in the viewpoint of the enhancements of the foam quality, and is preferably 20 mPa·s or less, more preferably 15 mPa·s or less, and further preferably 10 mPa·s or less, in the viewpoint of the dischargeability of foam. More specifically, the range of the viscosity at 30°C of the hair cosmetic composition is preferably 0.8 to 20 mPa·s, more preferably 1 to 15 mPa·s, further preferably 2 to 10 mPa·s, and still further preferably 3 to 10 mPa·s.

The viscosity at 30°C can be specifically measured by the method described in the examples.

### <Viscosity at 5°C>

The viscosity at 5°C of the hair cosmetic composition is preferably 1.5 mPa·s or more, more preferably 2 mPa·s or more, further preferably 4 mPa·s or more, and still further preferably 6 mPa·s or more, in the viewpoints of the enhancements of the foam quality, and is preferably 50 mPa·s or less, more preferably 30 mPa·s or less, further preferably 20 mPa·s or less, and still further preferably 15 mPa·s or less, in the viewpoint of the dischargeability of foam. More specifically, the range of the viscosity at 5°C of the hair cosmetic composition is preferably 1.5 to 50 mPa·s, more preferably 2 to 30 mPa·s, further preferably 4 to 20 mPa·s, and still further preferably 6 to 15 mPa·s.

The viscosity at 5°C can be specifically measured by the method described in the examples.

### (Method of Producing Hair Cosmetic Composition)

The method of producing the hair cosmetic composition is not particularly limited. For example, the hair cosmetic composition can be produced by blending the components (A) to (C) and additional components used depending on necessity by the method described in Examples, and mixing with known agitation equipment.

### [Hair Cosmetics]

The hair cosmetic composition of the present invention is preferably used as hair cosmetics filled up a non-gas type foam discharge container. Examples of the hair cosmetics include a hair cleanser, such as a shampoo, a hair rinse, a hair conditioning agent, a hair treatment agent (including one that is not washed off), a hair styling agent, a hair dye, and a hair growth agent. Among these, the hair cosmetics are preferably a hair rinse, a hair conditioning agent, a hair treatment agent, or a hair styling agent, and more preferably a hair conditioning agent.

Any type of non-gas type foam discharge containers may be used as far as the charged hair cosmetic composition is mixed with air in the discharge container, and is extruded in the form of foam. Examples thereof include a pump foamer container, in which the pump head is pushed to pressurize the cylinder of the air chamber and the cylinder of the hair cosmetic composition, and thereby the air and the hair cosmetic composition are mixed and discharged in the form of foam, and a squeeze foamer container, in which the body of the press-deformable container is pushed to deform the container for discharging.

A porous membrane filter, such as a mesh, is preferably provided on the discharge flow channel for the hair cosmetic composition of the foam discharge container in the viewpoint of controlling of the foam quality.

Specific examples of the non-gas type foam discharge container include the foamer containers produced by Yoshinokogyosho Co., Ltd. and Daiwa Can Company. The foam discharge containers described in JP 7-315463 A, JP 8-230961 A, JP 2005-193972 A, and the like may also be used.

### (Using Method of Hair Cosmetics)

The using method of the hair cosmetics is not particularly limited as far as the method is a method of applying the hair cosmetics formed into foam by a foam discharge container to the hair. In the method of applying to the hair, the hair cosmetics in the form of foam discharged from the foam discharge container may be applied to either the wet hair or the dry hair, but are preferably applied to the wet hair.

In the case where the hair cosmetics are a hair rinse, a hair conditioning agent, or a hair treatment agent, examples of the using method include a method of applying and fitting the hair cosmetics in the form of foam discharged from the foam discharge container to the hair after cleansing the hair, and then washed off depending on necessity. In the case where the hair cosmetics are a hair cleanser, examples of the using method include a method of cleansing the hair with the hair cosmetics in the form of foam discharged from the foam discharge container. The cleansing method includes, for example, applying the hair cosmetics in the form of foam discharged from the foam discharge container to the hair, and washing off the hair cosmetics. The cleansing method preferably includes applying the hair cosmetics in the form of foam discharged from the foam discharge container to the hair, and washing off the hair cosmetics, and more preferably includes applying the hair cosmetics in the form of foam discharged from the foam discharge container to the hair, further foaming the applied hair cosmetics, and then washing off the hair cosmetics. By applying the foamed hair cosmetics to the hair, the hair cosmetics can be readily spread to the entire hair, and can be sufficiently fit to the hair.

By using the hair cosmetics of the present invention, for example, by the aforementioned method, clogging of the foam discharge container can be prevented even under a low temperature environment. By the virtue of the foam having a favorable quality in application, the hair can be protected against damages caused by friction of the hair. The smoothness can rest on the hair and suppress the entanglement of the hair during time of rinsing.

The amount of the hair cosmetics to be applied to the hair in terms of mass ratio to the mass of the hair (mass of hair cosmetics/mass of hair) is preferably 0.02 or more, more preferably 0.03 or more, and further preferably 0.05 or more, and is preferably 0.6 or less, and more preferably 0.5 or less. It will suffice to treat at least a part of the head hair.

With respect to the aforementioned embodiments, the present invention relates to the following hair cosmetic compositions and hair cosmetics.
<1> A hair cosmetic composition
   containing or being prepared by blending a component (A), a component (B), and a component (C) below:
   component (A): one or more kinds of a cationic surfactant selected from the group consisting of an alkyltrimethylammonium salt and an alkylamidoalkyltrimethylammonium salt,
   component (B): a betaine type surfactant, and
   component (C): one or more kinds selected from the group consisting of one or more kinds of a nonionic surfactant (C1) selected from the group consisting of a polyoxyalkylene alkyl ether, an alkyl glucoside, an alkyl alkanolamide, and an alkyl glyceryl ether, one or more kinds of a modified silicone having a cationic group (C2) selected from the group consisting of an amino polyether-modified silicone and a quaternary ammonium cation-modified silicone, and a sugar alcohol having 4 or more carbon atoms (C3),
   having a mass ratio [(B)/(A)] of a content or a blending amount of the component (B) to a content or a blending amount of the component (A) of 0.4 or more and 5 or less,
   having a content or a blending amount of a higher alcohol of 0.1% by mass or less, and
   being used by filling up a non-gas type foam discharge container.
<2> The hair cosmetic composition according to the item <1>, wherein the amino polyether-modified silicone has a structure represented by the following general formula (1-2): 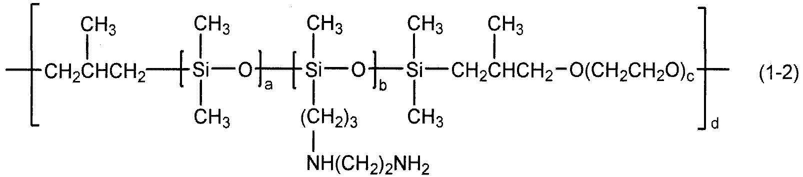 wherein in the formula (1-2), a represents a number of 2 or more; b represents a number of 1 or more; c represents a number of 4 or more and 100 or less; and d represents a number of 1 or more, provided that the structural units in brackets are not restricted in bonding order and may have any of a block bonding mode and a random bonding mode, and the c number of groups represented by CₙH₂ₙO may be the same as or different from each other.
<3> The hair cosmetic composition according to the item <1> or <2>, wherein the quaternary ammonium cation-modified silicone has a structure represented by the following general formula (2-3): 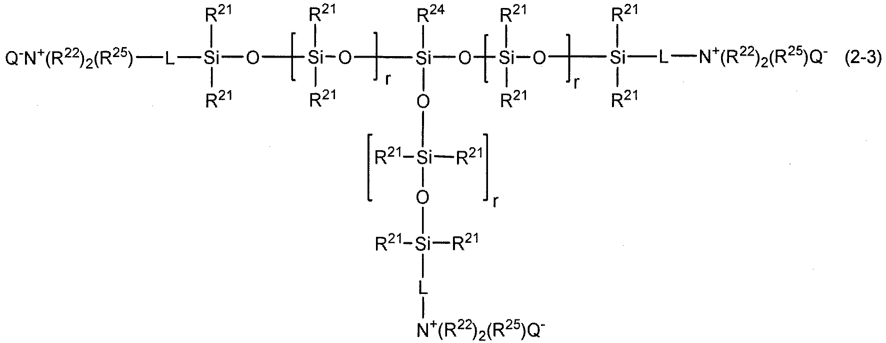 wherein in the general formula (2-3), R²¹ represents a monovalent hydrocarbon group having 1 or more and 6 or less carbon atoms; R²² represents a hydrogen atom, a hydrocarbon group having 1 or more and 20 or less carbon atoms, or a hydrocarbon group containing an amide bond having 1 or more and 20 or less carbon atoms; L represents a divalent organic group; Q⁻represents a counter ion of the quaternary ammonium ion; and r represents a number of 2 or more, provided that the structural units in brackets are not restricted in bonding order and may have any of a block bonding mode and a random bonding mode, the plural groups represented by each of R²¹ and R²² may be the same as or different from each other; R²⁴ represents an alkyl group having 1 or more and 6 or less carbon atoms or a phenyl group; and R²⁵ represents a hydrocarbon group having 1 or more and 20 or less carbon atoms or a hydrocarbon group containing an amide bond having 1 or more and 20 or less carbon atoms.
<4> The hair cosmetic composition according to any one of the items <1> to <3>, wherein the component (C3) is one or more kinds selected from the group consisting of erythritol and mannitol.
<5> The hair cosmetic composition according to any one of the items <1> to <4>, wherein the component (C) contains the component (C1), and the mass ratio (((A)+(B))/(C1)) of the total content or the total blending amount of the component (A) and the component (B) to the content or the blending amount of the component (C1) is 0.1 or more and 25 or less.
<6> The hair cosmetic composition according to any one of the items <1> to <5>, wherein the component (C) contains the component (C1), and the mass ratio (((A)+(B))/(C1)) of the total content or the total blending amount of the component (A) and the component (B) to the content or the blending amount of the component (C1) is 3 or more and 10 or less.
<7> The hair cosmetic composition according to the item <5> or <6>, wherein the component (C) further contains the component (C2).
<8> The hair cosmetic composition according to the item <7>, wherein the mass ratio [(C2)/(C1)] of the content or the blending amount of the component (C2) to the content or the blending amount of the component (C1) is 0.08 or more and 10 or less.
<9> The hair cosmetic composition according to any one of the items <5> to <8>, wherein the content or the blending amount of the component (C1) is 0.08% by mass or more and 6% by mass or less.
<10> The hair cosmetic composition according to any one of the items <5> to <9>, wherein the content or the blending amount of the component (C1) is 0.30% by mass or more and 2% by mass or less.
<11> The hair cosmetic composition according to any one of the items <1> to <4>, wherein the component (C) contains one or more kinds selected from the group consisting of the component (C2) and the component (C3).
<12> The hair cosmetic composition according to the item <11>, wherein the mass ratio (((A)+(B))/((C2)+(C3))) of the total content or the total blending amount of the component (A) and the component (B) to the total content or the total blending amount of the component (C2) and the component (C3) is 0.1 or more and 40 or less.
<13> The hair cosmetic composition according to the item <11> or <12>, wherein the mass ratio (((A)+(B))/((C2)+(C3))) of the total content or the total blending amount of the component (A) and the component (B) to the total content or the total blending amount of the component (C2) and the component (C3) is 1 or more and 15 or less.
<14> The hair cosmetic composition according to any one of the items <11> to <13>, wherein the total content or the total blending amount of the component (C2) and the component (C3) is 0.1% by mass or more and 5% by mass or less.
<15> The hair cosmetic composition according to any one of the items <11> to <14>, wherein the total content or the total blending amount of the component (C2) and the component (C3) is 0.5% by mass or more and 2% by mass or less.
<16> The hair cosmetic composition according to any one of the items <1> to <15>, wherein the content or the blending amount of the component (A) is 0.08% by mass or more and 5% by mass or less.
<17> The hair cosmetic composition according to any one of the items <1> to <16>, wherein the content or the blending amount of the component (A) is 0.50% by mass or more and 3% by mass or less.
<18> The hair cosmetic composition according to any one of the items <1> to <17>, wherein the content or the blending amount of the component (B) is 0.08% by mass or more and 15% by mass or less.
<19> The hair cosmetic composition according to any one of the items <1> to <18>, wherein the content or the blending amount of the component (B) is 1% by mass or more and 5% by mass or less.
<20> The hair cosmetic composition according to any one of the items <1> to <19>, wherein the total content or the total blending amount of the component (A), the component (B), and the component (C) is 0.2% by mass or more and 20% by mass or less.
<21> The hair cosmetic composition according to any one of the items <1> to <20>, wherein the total content or the total blending amount of the component (A), the component (B), and the component (C) is 1% by mass or more and 10% by mass or less.
<22> The hair cosmetic composition according to any one of the items <1> to <21>, wherein the hair cosmetic composition further contains or is prepared by further blending a component (D) below:
   component (D): an anionic surfactant.
<23> The hair cosmetic composition according to the item <22>, wherein the content or the blending amount of the component (D) is 0.02% by mass or more and 0.1% by mass or less.
<24> The hair cosmetic composition according to any one of the items <1> to <23>, wherein the hair cosmetic composition further contains or is prepared by further blending a cationic polymer.
<25> The hair cosmetic composition according to the item <24>, wherein the cationic polymer is one or more kinds selected from the group consisting of cationized guar gum, a cationized hydroxyalkyl cellulose, and a quaternized dialkylaminoalkyl (meth)acrylate polymer.
<26> The hair cosmetic composition according to the item <24> or <25>, wherein the content or the blending amount of the cationic polymer is 0.1% by mass or more and 0.5% by mass or less.
<27> The hair cosmetic composition according to any one of the items <1> to <26>, wherein the hair cosmetic composition further contains or is prepared by further blending an organic acid or an inorganic acid.
<28> The hair cosmetic composition according to the item <27>, wherein the content or the blending amount of the organic acid or inorganic acid is 0.02% by mass or more and 0.5% by mass or less.
<29> The hair cosmetic composition according to any one of the items <1> to <28>, wherein the hair cosmetic composition further contains or is prepared by further blending an organic acid, and the organic acid is a hydroxycarboxylic acid.
<30> The hair cosmetic composition according to any one of the items <1> to <29>, wherein the hair cosmetic composition has a viscosity at 30°C of 0.8 mPa·s or more and 20 mPa·s or less.
<31> The hair cosmetic composition according to any one of the items <1> to <30>, wherein the hair cosmetic composition has a viscosity at 30°C of 3 mPa·s or more and 10 mPa·s or less.
<32> The hair cosmetic composition according to any one of the items <1> to <31>, wherein the hair cosmetic composition has a viscosity at 5°C of 1.5 mPa·s or more and 50 mPa·s or less.
<33> The hair cosmetic composition according to any one of the items <1> to <33>, wherein the hair cosmetic composition has a viscosity at 5°C of 6 mPa·s or more and 15 mPa·s or less.
<34> Hair cosmetics including the hair cosmetic composition according to any one of the items <1> to <33> filled up a non-gas type foam discharge container.

### EXAMPLES

The present invention will be described with reference to examples below, but the present invention is not limited to the range of the examples. The following measurements were carried out on the examples.

### (Measurement of pH)

The hair cosmetic composition was diluted by purified water for 5% aqueous solution. The pH thereof was measured by a pH meter (F-51, produced by Horiba, Ltd.) at 25°C.

### (Measurement of Viscosity: 5°C)

Approximately 100 mL of each of the hair cosmetic compositions filled in a 110 mL glass bottle, which was then sealed and stored in a thermostatic chamber at 5°C for 24 hours or more. Thereafter, the viscosity of the example was measured by a BM type viscometer using No. 1 rotor (TV-10, produced by Toki Sangyo Co., Ltd.) at rotational speed of 60 rpm after starting for 60 seconds, which the record of the viscosity value was taken and therefore designated as the viscosity at 5°C of the hair cosmetic composition.

### (Measurement of Viscosity: 30°C)

Approximately 100 mL of each of the hair cosmetic compositions filled in a 110 mL glass bottle, which was then sealed and stored in a thermostatic chamber at 30°C for 1 hour or more. Thereafter, the viscosity of the example was measured by a BM type viscometer using No. 1 rotor (TV-10, produced by Toki Sangyo Co., Ltd.) at rotational speed of 60 rpm after starting for 60 seconds, which the record of the viscosity value was taken and therefore designated as the viscosity at 30°C of the hair cosmetic composition.

### Examples 1 to 29 and Comparative Examples 1 to 10

### (Preparation and Evaluation of Hair Cosmetic Compositions)

Samples of the hair cosmetic compositions were prepared as examples of hair conditioning compositions according to Tables 2 to 6. The following measurements and evaluations were carried out on the examples for the hair cosmetic compositions or hair cosmetics including the hair cosmetic compositions filled up the foam discharge container described later. The results thereof are shown in Tables 2 to 6.

The blending amounts shown in Tables 2 to 6 were the effective amounts (% by mass) of the components.

The blending amounts of the organic solvents shown in Tables 2 to 6 were controlled by adding dipropylene glycol to the organic solvents derived from the raw materials of the hair cosmetic compositions.

The components used in Tables 2 to 6 are shown in Table 1.

**Table 1**

| Trade name of raw material | Manufacturer | Displayed name or component name |
|---|---|---|
| VARISOFT PATC | Evonik Japan, Co., Ltd. | Palmitamidopropyltrimonium chloride (palmitamidopropyl trimethylammonium chloride) |
| Quartamin 60W | Kao Corporation | Cetrimonium chloride (cetyltrimethylammonium chloride) |
| | | Water |
| Quartamin 86P conc | Kao Corporation | Steartrimonium chloride (stearyltrimethylammonium chloride) |
| | | Isopropyl alcohol |
| | | Water |
| Amphitol 20HD | Kao Corporation | Lauryl hydroxysultine (lauryl hydroxysulfo betaine) |
| | | Water |
| Amphitol 20AB | Kao Corporation | Lauramidopropyl betaine (lauric acid amidopropyl betaine) |
| | | Water |
| Amphitol 20Y-B | Kao Corporation | Sodium cocoamphoacetate (2-alkyl-N-carboxymethyl-N-hydroxyethylimidazolinium betaine) |
| | | Ethanol |
| | | Water |
| Kao Sofcare GP-1 | Kao Corporation | PPG-3 caprylyl ether |
| Penetol GE-ID | Kao Corporation | Isodecyl glyceryl ether |
| Aminone C-11 S | Kao Corporation | Cocamide methyl MEA (coconut oil fatty acid N-methylethanolamide) |
| Emulgen 116 | Kao Corporation | Laureth-16 (PEG-16 lauryl ether) |
| AG-124 | Kao Corporation | Lauryl qlucoside |
| DOWSIL SS-3588 | Dow Toray Co., Ltd. | (Bisisobutyl PEG-15/amodimethicone) copolymer |
| | | Ethanol |
| ABIL ME45 | Evonik Japan, Co., Ltd. | Silicone quaternium-22 |
| | | Polyglyceryl-3 caprate |
| | | Dipropylene glycol |
| | | Cocamidopropyl betaine (coconut oil fatty acid amidopropyl dimethylaminoacetate betaine) |
| | | Palmitamidopropyltrimonium chloride (palmitamidopropyl trimethylammonium chloride) |
| | | Propylene glycol |
| | | Water |
| Zerose Erythritol STD GRAN | Cargill, Inc. | Erythritol |
| Marine Crystal | Mitsubishi Corporation Life Sciences Ltd. | Mannitol |
| Kalcol 8098 | Kao Corporation | Stearyl alcohol |
| Kalcol 4098 | Kao Corporation | Myristvl alcohol |
| EMAL 227-PH 11 | Kao Corporation | Sodium laureth sulfate (sodium polyoxyethylene lauryl ether sulfate) |
| Caticello H-60 | Kao Corporation | Polyquaternium-10 (cationized cellulose) |

### (Storage Stability under Low Temperature (-5°C) Environment (Low Temperature Stability))

Approximately 100 mL of each of the hair cosmetic compositions filled in a 110 mL glass bottle, which was then sealed and stored in a thermostatic chamber at -5°C for 3 days, and the appearance thereof was visually inspected and graded according to the following evaluation standard.

### [Evaluation Standard]

A: transparent
B: translucent
C: white turbidity, or deposition, separation, or precipitation observed

### (Evaluation of Foam Quality Discharged from Foam Discharge Container: Room Temperature)

Approximately 100 mL of each of the hair cosmetic compositions filled in a foam discharge container having a pump head for hand pressing to discharge foam. The foam discharge container is "Pump Foamer", produced by Yoshinokogyosho Co., Ltd., mesh size of porous filter disposed on foam discharge flow channel: #200, number of plies of mesh: 2 plies, liquid discharge amount of each pump: approximately 1 g, air discharge amount (foam discharge amount) of each pump: approximately 13 cm³, capacity of container: approximately 300 mL. Then the foam discharge container was sealed and kept still. To discharge foam in one pump, the foam discharge container was placed on a flat surface, and an operation of discharging foam was performed by pressing the pump head toward the said surface once. The discharged foam from the operation was visually inspected and graded according to the following evaluation standard by one expert panelist. Fig. 1 is a set of photographs showing the state of foam of the following evaluation standard.

### [Evaluation Standard]

A: firm foam
B: loose foam
C: large bubbles mixed in

### (Evaluation for Feeling Protective of Hair during Application)

A Japanese female hair bundle for the evaluation having a length of 30 cm, a width of 6 cm, and a weight of 20 g was sufficiently wetted with warm water at 35 to 40°C, and then washed with a plain shampoo, which is to be described thereafter. After squeezing water out of the washed hair, one pump of each of the hair cosmetics prepared in the examples was applied to the hair bundle, and the protective feeling of the hair was evaluated by performing sensory evaluation of the hair in fitting the hair cosmetics to the hair by seven panelists.

To give assessment, one of the following grades A, B, C was selected, A: The hair cosmetics was fit for the hair, made the hair feels smooth significantly, and gave feeling protective of the hair; B: Unclear to judge; C: The hair cosmetics was unfit for the hair, failing in the smoothness, and gave no feeling protective of the hair. Of Tables 2 to 6, the numerals placed in order from left to right are the numbers of panelists giving grades A, B, C, respectively.

### [Composition of Plain Shampoo]

| Components | (% by mass) |
|---|---|
| Sodium Polyoxyethylene (2.0) lauryl ether sulfate | 15.5 |
| Lauric acid diethanolamide | 2.2 |
| Disodium edetate | 0.15 |
| Sodium benzoate | 0.18 |
| Oxybenzone | 0.03 |
| Phosphoric acid | 0.07 |
| Sodium chloride | 0.8 |
| Fragrance | 0.4 |
| Purified water | balance |
| Total | 100.0 |

### (Evaluation of Feeling of Smoothness and Difficulty of Entanglement of Hair During Rinsing)

A Japanese female hair bundle for the evaluation having a length of 30 cm, a width of 6 cm, and a weight of 20 g was sufficiently wetted with warm water at 35 to 40°C, and then washed with the aforementioned plain shampoo. After squeezing water out of the washed hair, one pump of each of the hair cosmetics prepared in the examples was applied to the hair bundle, and after the hair cosmetics applied evenly onto the hair, a sensory evaluation was performed to assess feeling of smoothness and difficulty in entanglement of the hair during rinsing off the hair cosmetics with warm water at a temperature of 35 to 40°C by seven panelists.

To give assessment, one of the following grades A, B, C was selected, A: The hair felt smooth significantly, and was suppressed entanglement; B: Unclear to judge; C: The hair felt not smooth and became entangled. Of Tables 2 to 6, the numerals placed in order from left to right are the numbers of panelists giving grades A, B, C, respectively.

**Table 2**

| | | | | Example | | | | | | |
|---|---|---|---|---|---|---|---|---|---|---|
| | | | | 1 | 2 | 3 | 4 | 5 | 6 | 7 |
| Blending composition of hair cosmetic composition (% by mass) | (A) | | Palmitamidopropyltrimonium chloride | 1.00 | | 0.30 | | | | |
| | | | Cetrimonium chloride | | 1.00 | 0.70 | 1.00 | 1.00 | 1.00 | 1.00 |
| | | | Steartrimonium chloride. | | | | | | | |
| | (B) | | Lauryl hydroxysultine | 2.70 | 2.70 | 2.70 | | 2.00 | 2.70 | 2.70 |
| | | | Lauramidopropyl betaine | | | | 2.70 | 0.70 | | |
| | | | Cocamidopropyl betaine | | | | | | | |
| | | | Sodium cocoamphoacetate | | | | | | | |
| | (C) | (C1) | PPG-3 caprylyl ether | 0.50 | 0.50 | 0.50 | 0.50 | 0.50 | | 0.25 |
| | | | Isodecyl glyceryl ether | | | | | | 0.50 | 0.25 |
| | | | Polyglyceryl-3 caprate | | | | | | | |
| | | | Cocamide methyl MEA | | | | | | | |
| | | | Laureth-16 | | | | | | | |
| | | | Lauryl qlucoside | | | | | | | |
| | | (C2) | (Bisisobutyl PEG-15/amodimethicone) copolymer | 0.60 | 0.60 | 0.60 | 0.60 | 0.60 | 0.60 | 0.60 |
| | | | Silicone quaternium-22 | | | | | | | |
| | | (C3) | Erythritol | | | | | | | |
| | | | Mannitol | | | | | | | |
| | Higher alcohol | | Stearyl alcohol | | | | | | | |
| | | | Myristyl alcohol | | | | | | | |
| | (D) | | Sodium laureth sulfate | | | | | | | |
| | Cationic polymer | | Polyquaternium-10 | 0.10 | 0.10 | 0.10 | 0.10 | 0.10 | 0.10 | 0.10 |
| | Lactic acid - | | | 0.36 | 0.36 | 0.36 | 0.36 | 0.36 | 0.36 | 0.36 |
| | Aqueous medium | Organic solvent | | 7.00 | 7.00 | 7.00 | 7.00 | 7.00 | 7.00 | 7.00 |
| | | Water | | balance | balance | balance | balance | balance | balance | balance |
| Mass ratio | [(B)/(A)] | | | 2.7 | 2.7 | 2.7 | 2.7 | 2.7 | 2.7 | 2.7 |
| Mass ratio (((A)+(b))/(C1)) | | | | 7.4 | 7.4 | 7.4 | 7.4 | 7.4 | 7.4 | 7.4 |
| Mass ratio (((A)+(B))/((C2)+(C3))) | | | | 6.2 | 6.2 | 6.2 | 6.2 | 6.2 | 6.2 | 6.2 |
| Total content ((A)+(B)+(C)) (% by mass) | | | | 4.80 | 4.80 | 4.80 | 4.80 | 4.80 | 4.80 | 4.80 |
| Content of component (A) (% by mass) | | | | 1.00 | 1.00 | 1.00 | 1.00 | 1.00 | 1.00 | 1.00 |
| Content of component (B) (% by mass) | | | | 2.70 | 2.70 | 2.70 | 2.70 | 2.70 | 2.70 | 2.70 |
| Content of component (C1) (% by mass) | | | | 0.50 | 0.50 | 0.50 | 0.50 | 0.50 | 0.50 | 0.50 |
| Total content ((2)+(C3)) (% by mass) | | | | 0.60 | 0.60 | 0.60 | 0.60 | 0.60 | 0.60 | 0.60 |
| pH | | | | 3.51 | 3.48 | 3.46 | 3.73 | 3.55 | 3.43 | 3.44 |
| Viscosity at 30°C (mPa·s) | | | | 4.6 | 4.4 | 4.6 | 4.7 | 4.5 | 4.8 | 4.9 |
| Viscosity at 5°C (mPa·s) | | | | 7.6 | 7.6 | 7.5 | 7.6 | 7.4 | 7.5 | 7.8 |
| Evaluation result | Low temperature stability | | | A | A | A | A | A | A | A |
| | Foam quality | | | A | A | A | A | A | A | A |
| | Protective feeling of hair in application (A/B/C) | | | 6/1/0 | 6/1/0 | 6/1/0 | 6/1/0 | 6/1/0 | 6/1/0 | 5/2/0 |
| | Smoothness and difficulty in entanglement of hair during rinsing (A/B/C) | | | 6/1/0 | 6/1/0 | 6/1/0 | 6/1/0 | 6/1/0 | 4/3/0 | 4/3/0 |

**Table 3**

| | | | | Example | | | | | | | |
|---|---|---|---|---|---|---|---|---|---|---|---|
| | | | | 8 | 9 | 10 | 11 | 12 | 13 | 14 | 15 |
| | (A) | | Palmitamidopropyltrimonium chloride | 0.01 | | | | | | | |
| | | | Cetrimonium chloride | 1.00 | 1.00 | 1.00 | 1.00 | 2.00 | 2.00 | 2.00 | 0.50 |
| | | | Steartrimonium chloride | | | | | | | | |
| | (B) | | Lauryl hydroxysultine | 2.70 | 2.70 | 2.70 | 1.00 | 1.00 | 10.00 | 8.00 | 0.50 |
| | | | Lauramidopropyl betaine | | | | | | | | |
| | | | Cocamidopropyl betaine | 0.04 | | | | | | | |
| | | | Sodium cocoamphoacetate | | | | | | | | |
| Blending composition of hair cosmetic composition (% by mass) | (C) | (C1) | PPG-3 caprylyl ether | 0.50 | 0.50 | 0.50 | 0.50 | 0.50 | 0.50 | 0.70 | 0.80 |
| | | | Isodecyl glyceryl ether | | | | | | | | 0.70 |
| | | | caprate | 0.10 | | | | | | | |
| | | | Cocamide methyl MEA | | | | | | | | |
| | | | Laureth-16 | | | | | | | | 3.50 |
| | | | Lauryl glucoside | | | | | | | | |
| | | (C2) | (Bisisobutyl PEG-15/amodimethicone) copolymer | 0.30 | | | 0.60 | 0.60 | 0.60 | 0.60 | 0.60 |
| | | | Silicone quaternium-22 | 0.30 | | | | | | | |
| | | (C3) | Erythritol | | 0.60 | 0.30 | | | | | |
| | | | Mannitol | | | 0.30 | | | | | |
| | Higher alcohol | | Stearyl alcohol | | | | | | | | |
| | | | Myristyl alcohol | | | | | | | | |
| | (D) | | Sodium laureth sulfate | | | | | | | | |
| | Cationic polymer | | Polyquaternium-10 | 0.10 | 0.10 | 0.10 | 0.10 | 0.10 | 0.10 | 0.10 | 0.10 |
| | Lactic acid | | | 0.36 | 0.36 | 0.36 | 0.36 | 0.36 | 0.36 | 0.36 | 0.36 |
| | Aqueous medium | Organic solvent | | 7.00 | 7.00 | 7.00 | 7.00 | 7.00 | 7.00 | 7.00 | 7.00 |
| | | Water | | balance | balance | balance | balance | balance | balance | balance | balance |
| Mass ratio [(B)/(A)] | | | | 2.7 | 2.7 | 2.7 | 1.0 | 0.5 | 5.0 | 4.0 | 1.0 |
| Mass ratio (((A)+(b))/(C1)) | | | | 6.3 | 7.4 | 7.4 | 4.0 | 6.0 | 24.0 | 14.3 | 0.2 |
| Mass ratio (((A)+(B))/((C2)+(C3))) | | | | 6.3 | 6.2 | 6.2 | 3.3 | 5.0 | 20.0 | 16.7 | 1.7 |
| Total content ((A)+(B)+(C)) (% by mass) | | | | 4.95 | 4.80 | 4.80 | 3.10 | 4.10 | 13.10 | 11.30 | 6.60 |
| Content of component (A) (% by mass) | | | | 1.01 | 1.00 | 1.00 | 1.00 | 2.00 | 2.00 | 2.00 | 0.50 |
| Content of component (B) (% by mass) | | | | 2.74 | 2.70 | 2.70 | 1.00 | 1.00 | 10.00 | 8.00 | 0.50 |
| Content of component (C1) (% by mass) | | | | 0.60 | 0.50 | 0.50 | 0.50 | 0.50 | 0.50 | 0.70 | 5.00 |
| Total content ((C2)+(C3)) (% by mass) | | | | 0.60 | 0.60 | 0.60 | 0.60 | 0.60 | 0.60 | 0.60 | 0.60 |
| pH | | | | 3.43 | 3.30 | 3.29 | 3.43 | 3.45 | 3.45 | 3.45 | 3.55 |
| Viscosity at 30°C (mPa·s) | | | | 4.6 | 4.6 | 4.7 | 5.8 | 5.8 | 5.2 | 5.0 | 7.6 |
| Viscosity at 5°C (mPa·s) | | | | 7.4 | 7.6 | 7.4 | 9.3 | 9.3 | 8.4 | 7.8 | 12.5 |
| Evaluation result | Low temperature stability | | | A | A | A | A | A | A | A | A |
| | Foam quality | | | A | A | A | A | A | A | A | A |
| | Protective feeling of hair in application (A/B/C) | | | 6/1/0 | 5/2/0 | 5/2/0 | 3/4/0 | 5/2/0 | 4/3/0 | 5/2/0 | 4/3/0 |
| | Smoothness and difficulty in entanglement of hair durinq rinsing (A/B/C) | | | 6/1/0 | 4/3/0 | 4/3/0 | 5/2/0 | 6/1/0 | 3/4/0 | 5/2/0 | 5/2/0 |

**Table 4**

| | | | | Example | | | | | |
|---|---|---|---|---|---|---|---|---|---|
| | | | | 16 | 17 | 18 | 19 | 20 | 21 |
| Blending composition of hair cosmetic composition (% by mass) | (A) | | Palmitamidopropyltrimonium chloride | | 0.30 | 0.30 | 0.30 | | |
| | | | Cetrimonium chloride | 2.40 | 0.70 | 0.70 | 0.70 | 0.10 | 2.00 |
| | | | Steartrimonium chloride. | | | | | | |
| | (B) | | Lauryl hydroxysultine | 3.60 | 2.00 | 2.00 | 2.00 | 0.10 | 6.00 |
| | | | Lauramidopropyl betaine | | 0.70 | 0.70 | 0.70 | | |
| | | | Cocamidopropyl betaine | | | | | | |
| | | | Sodium cocoamphoacetate | | | | | | |
| | (C) | (C1) | PPG-3 caprylyl ether | 0.50 | 0.50 | | | 0.10 | 0.50 |
| | | | Isodecyl glyceryl ether | | | | | | 0.50 |
| | | | Polyqlyceryl-3 caprate | | | | | | |
| | | | Cocamide methyl MEA | | | | | | |
| | | | Laureth-16 | | | | | | 3.00 |
| | | | Lauryl glucoside | | 0.20 | | | | |
| | | (C2) | (Bisisobutyl PEG-15/amodimethicone) copolymer | 0.60 | | | 0.60 | 0.60 | 0.60 |
| | | | Silicone quaternium-22 | | | | | | |
| | | (C3) | Erythritol | | | 0.60 | | | |
| | | | Mannitol | | | 0.60 | | | |
| | Higher alcohol | | Stearyl alcohol | | | | | | |
| | | | Myristyl alcohol | | | | | | |
| | (D) | | Sodium laureth sulfate | | | | | | |
| | Cationic polymer | | Polyquaternium-10 | 0.10 | 0.10 | 0.10 | 0.10 | 0.10 | 0.10 |
| | Lactic acid | | | 0.36 | 0.36 | 0.36 | 0.36 | 0.36 | 0.36 |
| | Aqueous medium | Organic solvent | | 7.00 | 7.00 | 7.00 | 7.00 | 7.00 | 7.00 |
| | | Water | | balance | balance | balance | balance | balance | balance |
| Mass ratio [(B)/(A)] | | | | 1.5 | 2.7 | 2.7 | 2.7 | 1.0 | 3.0 |
| Mass ratio (((A)+(B))/(C1)) | | | | 12.0 | 5.3 | - | - | 2.0 | 2.0 |
| Mass ratio (((A)+(B))/((C2)+(C3))) | | | | 10.0 | - | 3.1 | 6.2 | 0.3 | 13.3 |
| Total content ((A)+(B)+(C)) (% by mass) | | | | 7.10 | 4.40 | 4.90 | 4.30 | 0.90 | 12.60 |
| Content of component (A) (% by mass) | | | | 2.40 | 1.00 | 1.00 | 1.00 | 0.10 | 2.00 |
| Content of component (B) (% by mass) | | | | 3.60 | 2.70 | 2.70 | 2.70 | 0.10 | 6.00 |
| Content of component (C1) (% by mass) | | | | 0.50 | 0.70 | 0.00 | 0.00 | 0.10 | 4.00 |
| Total content ((C2)+(C3)) (% by mass) | | | | 0.60 | 0.00 | 1.20 | 0.60 | 0.60 | 0.60 |
| pH | | | | 3.46 | 3.40 | 3.40 | 3.62 | 3.82 | 3.47 |
| Viscosity at 30°C (mPa·s) | | | | 5.2 | 4.6 | 4.6 | 4.8 | 5.3 | 7.3 |
| Viscosity at 5°C (mPa·s) | | | | 7.9 | 8.0 | 8.0 | 7.7 | 7.8 | 11.4 |
| Evaluation result | Low temperature stability | | | A | A | A | A | A | A |
| | Foam quality- | | | A | A | A | A | B | A |
| | Protective feeling of hair in application (A/B/C) | | | 5/2/0 | 3/4/0 | 5/2/0 | 5/2/0 | 4/3/0 | 5/2/0 |
| | Smoothness and difficulty in entanglement of hair during rinsing (A/B/C) | | | 5/2/0 | 4/3/0 | 4/3/0 | 5/2/0 | 5/2/0 | 5/2/0 |

**Table 5**

| | | | | Example | | | | | | | |
|---|---|---|---|---|---|---|---|---|---|---|---|
| | | | | 22 | 23 | 24 | 25 | 26 | 27 | 28 | 29 |
| | (A) | | Palmitamidopropyltrimonium chloride | 0.30 | 0.50 | 0.31 | | | | | |
| | | | Cetrimonium chloride | 0.70 | 0.90 | 0.70 | 2.00 | 1.00 | 2.40 | 2.00 | 2.00 |
| | | | Steartrimonium chloride | | | | | 1.00 | | | |
| | (B) | | Lauryl hydroxysultine | 2.00 | 2.00 | 2.00 | 1.50 | 1.50 | 8.00 | 6.00 | 1.50 |
| | | | Lauramidopropyl betaine | 0.70 | 1.00 | 0.70 | 2.00 | 2.00 | | | 2.00 |
| | | | Cocamidopropyl betaine | | | 0.04 | | | | | |
| | | | Sodium cocoamphoacetate | | | | 0.50 | 0.50 | | | 0.50 |
| Blending composition of hair cosmetic composition (% by mass) | (C) | (C1) | PPG-3 caprylyl ether | 0.50 | | 0.50 | 0.25 | 0.50 | 0.70 | 0.50 | 0.50 |
| | | | Isodecyl glyceryl ether | | 0.50 | | 0.25 | | | 0.50 | |
| | | | Polyglyceryl-3 caprate | | | 0.10 | | | | | |
| | | | Cocamide methyl MEA | | | | 0.25 | | | | |
| | | | Laureth-16 | | | | 0.75 | 1.00 | | 3.00 | 1.00 |
| | | | Lauryl glucoside | | | | | | | | |
| | | (C2) | (Bisisobutyl PEG-15/amodimethicone) copolymer | 0.60 | 0.60 | 0.30 | 0.60 | 0.18 | 0.60 | 0.60 | 0.18 |
| | | | Silicone quaternium-22 | | | 0.30 | | | | | |
| | | (C3) | Erythritol | | | 0.60 | | | | | |
| | | | Mannitol | | | 0.60 | | | | | |
| | Higher alcohol | | Stearyl alcohol | | | | | | | | |
| | | | Myristyl alcohol | | | | | | 0.10 | | |
| | (D) | | Sodium laureth sulfate | | | | | | | 0.05 | 0.03 |
| | Cationic polymer | | Polyquaternium-10 | 0.10 | 0.10 | 0.10 | 0.10 | 0.10 | 0.10 | 0.10 | 0.10 |
| | Lactic acid | | | 0.36 | 0.36 | 0.36 | 0.36 | 0.36 | 0.36 | 0.36 | 0.36 |
| | Aqueous medium | Organic solvent | | 7.00 | 7.00 | 7.00 | 7.00 | 7.00 | 7.00 | 7.00 | 7.00 |
| | | Water | | balance | balance | balance | balance | balance | balance | balance | balance |
| Mass ratio [(B)/(A)1 | | | | 2.7 | 2.1 | 2.7 | 2.0 | 2.0 | 3.3 | 3.0 | 2.0 |
| Mass ratio (((A)+(B))/(C1)) | | | | 7.4 | 8.8 | 6.3 | 4.0 | 4.0 | 14.9 | 2.0 | 4.0 |
| Mass ratio (((A)+(B))/((C2)+(C3))) | | | | 6.2 | 7.3 | 2.1 | 10.0 | 33.3 | 17.3 | 13.3 | 33.3 |
| Total content ((A)+(B)+(C)) (% by mass) | | | | 4.80 | 5.50 | 6.15 | 8.10 | 7.68 | 11.70 | 12.60 | 7.68 |
| Content of component (A) (% by mass) | | | | 1.00 | 1.40 | 1.01 | 2.00 | 2.00 | 2.40 | 2.00 | 2.00 |
| Content of component (B) (% by mass) | | | | 2.70 | 3.00 | 2.74 | 4.00 | 4.00 | 8.00 | 6.00 | 4.00 |
| Content of component (C1) (% by mass) | | | | 0.50 | 0.50 | 0.60 | 1.50 | 1.50 | 0.70 | 4.00 | 1.50 |
| Total content (((2)+(C3)) (% by mass) | | | | 0.60 | 0.60 | 1.80 | 0.60 | 0.18 | 0.60 | 0.60 | 0.18 |
| pH | | | | 3.54 | 3.50 | 3.48 | 4.10 | 3.98 | 3.43 | 3.62 | 3.78 |
| Viscosity at 30°C (mPa·s) | | | | 5.4 | 5.0 | 4.5 | 6.8 | 5.0 | 5.6 | 6.8 | 5.0 |
| Viscosity at 5°C (mPa·s) | | | | 7.4 | 7.4 | 7.4 | 13.1 | 7.6 | 8.9 | 11.5 | 8.4 |
| Evaluation result | Low temperature stability | | | A | A | A | A | A | A | A | A |
| | Foam quality | | | A | A | A | A | A | A | A | A |
| | Protective feeling of hair in application (A/B/C) | | | 7/0/0 | 7/0/0 | 7/0/0 | 7/0/0 | 6/1/0 | 6/1/0 | 6/1/0 | 7/0/0 |
| | Smoothness and difficulty in entanglement of hair during rinsing (A/B/C) | | | 7/0/0 | 6/1/0 | 7/0/0 | 7/0/0 | 6/1/0 | 4/3/0 | 6/1/0 | 6/1/0 |

**Table 6**

| | | | | Comparative Example | | | | | | | | | |
|---|---|---|---|---|---|---|---|---|---|---|---|---|---|
| | | | | 1 | 2 | 3 | 4 | 5 | 6 | 7 | 8 | 9 | 10 |
| | (A) | | Palmitamidopropyltrimonium chloride | | | | | | | | | | 0.40 |
| | | | Cetrimonium chloride | | 0.001 | 0.01 | 0.10 | 1.00 | 1.00 | 1.00 | 8.00 | 2.00 | |
| | | | Steartrimonium chloride | | | | | | | | | | |
| | (B) | | Lauryl hydroxysultine | 2.70 | 2.70 | 2.70 | 2.70 | | 2.70 | 2.70 | | | 2.70 |
| | | | Lauramidopropyl betaine | | | | | | | | 2.00 | 8.00 | |
| | | | Cocamidopropyl betaine | | | | | | | | | | |
| | | | Sodium cocoamphoacetate | | | | | | | | | | |
| Blending composition of hair cosmetic composition (% by mass) | (C) | (C1) | PPG-3 caprylyl ether | 0.50 | 0.50 | 0.50 | 0.50 | 0.50 | | 0.50 | | | 0.50 |
| | | | Isodecyl glyceryl ether | | | | | | | | | | |
| | | | Polyglyceryl-3 caprate | | | | | | | | | | |
| | | | Cocamide methyl MEA | | | | | | | | | | |
| | | | Laureth-16 | | | | | | | | 0.10 | 0.10 | |
| | | | Lauryl glucoside | | | | | | | | | | |
| | | (C2) | (Bisisobutyl PEG-15/amodimethicone) copolymer | 0.60 | 0.60 | 0.60 | 0.60 | 0.60 | | 0.60 | 0.60 | 0.60 | 0.60 |
| | | | Silicone quaternium-22 | | | | | | | | | | |
| | | (C3) | Erythritol | | | | | | | | | | |
| | | | Mannitol | | | | | | | | | | |
| | Higher alcohol | | Stearyl alcohol | | | | | | | 0.80 | | 0.30 | |
| | | | Myristyl alcohol | | | | | | | | | | |
| | (D) | | Sodium laureth sulfate | | | | | | | | | | |
| | Cationic polymer | | Polyquaternium-10 | 0.10 | 0.10 | 0.10 | 0.10 | 0.10 | 0.10 | 0.10 | 0.10 | 0.10 | 0.10 |
| | Lactic acid | | | 0.36 | 0.36 | 0.36 | 0.36 | 0.36 | 0.36 | 0.36 | 0.36 | 0.36 | 0.36 |
| | Aqueous medium | Organic solvent | | 7.00 | 7.00 | 7.00 | 7.00 | 7.00 | 7.00 | 7.00 | 7.00 | 7.00 | 7.00 |
| | | Water | | balance | balance | balance | balance | balance | balance | balance | balance | balance | balance |
| Mass ratio | ([B)/(A)] | | | - | 2700 | 270.0 | 27.0 | 0.0 | 2.7 | 2.7 | 0.3 | 4.0 | 6.8 |
| Mass ratio (((A)+(B))/(C1)) | | | | 5.4 | 5.4 | 5.4 | 5.6 | 2.0 | - | 7.4 | 100.0 | 100.0 | 6.2 |
| Mass ratio (((A)+(B))/((C2)+(C3))) | | | | 4.5 | 4.5 | 4.5 | 4.7 | 1.7 | - | 6.2 | 16.7 | 16.7 | 5.2 |
| Total content ((A)+(B)+(C)) (% by mass) | | | | 3.80 | 3.80 | 3.81 | 3.90 | 2.10 | 3.70 | 4.80 | 10.70 | 10.70 | 4.20 |
| Content of component (A) (% by mass) | | | | 0.000 | 0.001 | 0.010 | 0.100 | 1.00 | 1.00 | 1.00 | 8.00 | 2.00 | 0.40 |
| Content of component (B) (% by mass) | | | | 2.70 | 2.70 | 2.70 | 2.70 | 0.00 | 2.70 | 2.70 | 2.00 | 8.00 | 2.70 |
| Content of component (C1) (% by mass) | | | | 0.50 | 0.50 | 0.50 | 0.50 | 0.50 | 0.00 | 0.50 | 0.10 | 0.10 | 0.50 |
| Total content ((C2)+(C3)) (% by mass) | | | | 0.60 | 0.60 | 0.60 | 0.60 | 0.60 | 0.00 | 0.60 | 0.60 | 0.60 | 0.60 |
| pH | | | | 3.46 | 3.46 | 3.50 | 3.46 | 3.40 | 3.34 | 3.47 | 3.58 | 4.17 | 3.50 |
| Viscosity at 30°C (mPa·s) | | | | 5.1 | 5.1 | 4.8 | 5.1 | 5.3 | 4.5 | - | 6.3 | 5.6 | 4.5 |
| Viscosity at 5°C (mPa·s) | | | | 7.3 | 7.3 | 7.5 | 7.3 | 7.6 | 7.3 | - | 13.8 | 11 | 7.3 |
| Evaluation result | Low temperature stability | | | C | C | C | C | A | A | C | C | C | A |
| | Foam quality | | | A | A | A | A | C | A | *1 | A | A | A |
| | Protective feeling of hair in application (A/B/C) | | | 3/1/3 | 4/1/2 | 3/1/3 | 2/2/3 | 0/3/4 | 2/1/4 | 0/1/6 | 2/4/1 | 1/4/2 | 2/3/2 |
| | Smoothness and difficulty in entanglement of hair during rinsing (A/B/C) | | | 0/2/5 | 0/2/5 | 1/2/4 | 1/2/4 | 1/2/4 | 0/0/7 | 0/0/7 | 4/2/1 | 1/2/4 | 1/3/3 |

| | | | | | | | | | | | | | |
|---|---|---|---|---|---|---|---|---|---|---|---|---|---|
| *1: The evaluation of the foam quality was unable due to clogging occurring in discharging. | | | | | | | | | | | | | |

As shown in Tables 2 to 6, it is understood that the hair cosmetic compositions of Examples 1 to 29 have excellence in the storage stability under the low temperature environment and the quality of foam discharged from a foam discharge container, can protect the hair against damages caused by friction during application, impart the favorable smoothness to the hair and suppress the entanglement of the hair during time of rinsing, as compared to Comparative Examples 1 to 10.

### INDUSTRIAL APPLICABILITY

According to the hair cosmetic composition of the present invention, clogging of the foam discharge container can be prevented even under a low temperature environment. The hair can be protected against damages by the foam having favorable quality during time of application, and the smoothness can be imparted to the hair and suppress entanglement of the hair during time of rinsing, of which hair benefits to lead the comfort.

## Claims

1. A hair cosmetic composition
comprising a component (A), a component (B), and a component (C) below:
component (A): a cationic surfactant,
component (B): an amphoteric surfactant, and
component (C): one or more kinds selected from the group consisting of a nonionic surfactant (C1), a modified silicone having a cationic group (C2), and a sugar alcohol having 4 or more carbon atoms (C3),
having a mass ratio [(B)/(A)] of a content of the component (B) to a content of the component (A) of 0.4 or more and 5 or less,
having a content of a higher alcohol of 0.1% by mass or less, and
being used by filling up a non-gas type foam discharge container.

2. The hair cosmetic composition according to claim 1, wherein the component (C) contains the component (C1), and a mass ratio (((A)+(B))/(C1)) of a total content of the component (A) and the component (B) to a content of the component (C1) is 0.1 or more and 25 or less.

3. The hair cosmetic composition according to claim 2, wherein the component (C) further contains the component (C2).

4. The hair cosmetic composition according to claim 2 or 3, wherein a content of the component (C1) is 0.08% by mass or more and 6% by mass or less.

5. The hair cosmetic composition according to claim 1, wherein the component (C) contains one or more kinds selected from the group consisting of the component (C2) and the component (C3).

6. The hair cosmetic composition according to claim 5, wherein a mass ratio (((A)+(B))/((C2)+(C3))) of a total content of the component (A) and the component (B) to a total content of the component (C2) and the component (C3) is 0.1 or more and 40 or less.

7. The hair cosmetic composition according to claim 5 or 6, wherein a total content of the component (C2) and the component (C3) is 0.1% by mass or more and 5% by mass or less.

8. The hair cosmetic composition according to any one of claims 1 to 7, wherein a total content of the component (A), the component (B), and the component (C) is 0.2% by mass or more and 20% by mass or less.

9. The hair cosmetic composition according to any one of claims 1 to 8, wherein the hair cosmetic composition has a viscosity at 30°C of 0.8 mPa·s or more and 20 mPa·s or less.

10. The hair cosmetic composition according to any one of claims 1 to 9, wherein the hair cosmetic composition has a viscosity at 5°C of 1.5 mPa·s or more and 50 mPa·s or less.

11. The hair cosmetic composition according to any one of claims 1 to 10, wherein a content of the component (A) is 0.08% by mass or more and 5% by mass or less.

12. The hair cosmetic composition according to any one of claims 1 to 11, wherein a content of the component (B) is 0.08% by mass or more and 15% by mass or less.

13. The hair cosmetic composition according to any one of claims 1 to 12, wherein the hair cosmetic composition further comprises a component (D) below:
component (D): an anionic surfactant.

14. Hair cosmetics comprising the hair cosmetic composition according to any one of claims 1 to 13 filled up a non-gas type foam discharge container.
